(19) Europäisches Patentamt
European Patent Office
Office européen des brevets

(11) Publication number: **0 430 127 A2**

# (12) EUROPEAN PATENT APPLICATION

(21) Application number: **90122523.5**

(51) Int. Cl.[5]: **C07D 213/75**, A01N 43/40

(22) Date of filing: **26.11.90**

(30) Priority: **28.11.89 JP 308439/89**

(43) Date of publication of application:
**05.06.91 Bulletin 91/23**

(84) Designated Contracting States:
**AT BE CH DE DK ES FR GB GR IT LI LU NL SE**
**Bulletin**

(71) Applicant: **ISHIHARA SANGYO KAISHA, Ltd.**
**3-22, Edobori 1-chome**
**Nishi-ku Osaka-shi Osaka-fu(JP)**

(72) Inventor: **Shigehara, Itaru, c/o Ishihara Sangyo Kaisha, Ltd.**
**Central Laboratory, 3-1, Nishishibukawa 2-chome**
**Kusatsu-shi, Shiga(JP)**
Inventor: **Komyoji, Terumasa, c/o Ishihara Sangyo Kaisha, Ltd**
**Central Laboratory, 3-1, Nishishibukawa 2-chome**
**Kusatsu-shi, Shiga(JP)**
Inventor: **Nakajima, Toshio, c/o Ishihara Sangyo Kaisha, Ltd.**
**Central Laboratory, 3-1, Nishishibukawa 2-chome**
**Kusatsu-shi, Shiga(JP)**
Inventor: **Ito, Keiichiro, c/o Ishihara Sangyo Kaisha, Ltd.**
**Central Laboratory, 3-1, Nishishibukawa 2-chome**
**Kusatsu-shi, Shiga(JP)**
Inventor: **Mitani, Shigeru, c/o Ishihara Sangyo Kaisha, Ltd.**
**Central Laboratory, 3-1, Nishishibukawa 2-chome**
**Kusatsu-shi, Shiga(JP)**

(74) Representative: **Hansen, Bernd, Dr. Dipl.-Chem. et al**
**Hoffmann, Eitle & Partner Patent- und Rechtsanwälte Arabellastrasse 4**
**W-8000 München 81(DE)**

## (54) Pyridylcarbamate compounds, process for preparing the same, and biocidal composition containing the same for controlling harmful organisms.

(57) Novel pyridylcarbamate compounds useful as biocides for controlling harmful organisms are disclosed, which are represented by formula (I):

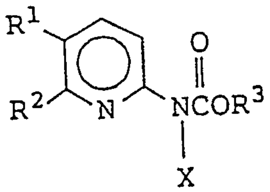

(I)

wherein $R^1$ represents a halogen atom, an alkoxy group, a trifluoromethyl group, a cyano group, or an alkylthio group; $R^2$ represents an alkyl group or an alkylthio group; $R^3$ represents a substituted or unsubstituted alkyl group, a substituted or unsubstituted alkenyl group, or a substituted or unsubstituted alkynyl group; and X represents a hydrogen atom, a substituted or unsubstituted alkyl group, or a substituted or unsubstituted acyl group; provided that a combination of $R^1$ representing a halogen atom, $R^2$ representing an alkyl group, $R^3$ representing an unsubstituted alkyl group or a tetrahalogenoethyl group, and X representing a hydrogen atom

EP 0 430 127 A2

and a combination of $R^1$ representing an alkoxy group and $R^2$ representing an alkylthio group are excluded.

# PYRIDYLCARBAMATE COMPOUNDS, PROCESS FOR PREPARING THE SAME, AND BIOCIDAL COMPOSITION CONTAINING THE SAME FOR CONTROLLING HARMFUL ORGANISMS

## FIELD OF THE INVENTION

This invention relates to a novel pyridylcarbamate compound, a process for preparing the same, and a biocidal composition containing the same for controlling harmful organisms.

## BACKGROUND OF THE INVENTION

Known pyridylcarbamate compounds relevant to the compounds according to the present invention are discussed below.

(1) Compounds represented by formula:

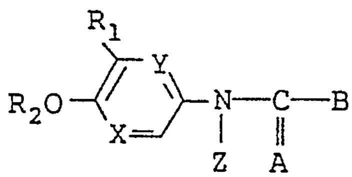

wherein one of X and Y represents a methine group, with the other representing a nitrogen atom; $R_1$ represents a halogen atom, an alkoxy group, an alkylthio group, etc.; $R_2$ represents an alkyl group, etc.; Z represents a hydrogen atom, an alkyl group, an alkylcarbonyl group, a cycloalkylcarbonyl group, etc.; A represents an oxygen atom or a sulfur atom; and B represents an alkoxy group which may be substituted with a halogen atom, a cyano group, an alkoxy group, etc., an alkenyloxy group which may be substituted with a halogen atom, an alkynyloxy group which may be substituted with a halogen atom, etc.,
are disclosed in European Patent 190,036A (corresponding to U.S. Patent 4,672,070). These compounds are different from the compounds of the present invention in position or kind of the substituent on the pyridine ring.

(2) Compounds represented by formula:

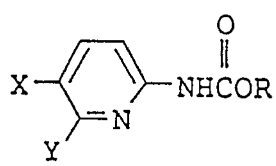

wherein X represents a halogen atom; Y represents a hydrogen atom, a methyl group, an ethyl group, or a propyl group; and R represents a lower alkyl group,
are disclosed in JP-A-55-35054, JP-A-55-36430, JP-A-55-40619, and JP-B-50-30694 (corresponding to U.S. Patents 3,914,240 and 3,969,362) (the terms "JP-A" and "JP-B" as used herein mean an "unexamined published Japanese patent application" and an "examined published Japanese patent application", respectively). These compounds are different from the compounds of the present invention in combination of the substituents on the pyridine ring and the carbamic ester moiety.

(3) Compounds represented by formula:

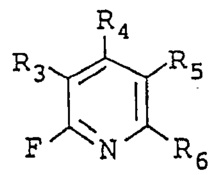

wherein $R_3$ and $R_5$ each represent a chlorine atom or a fluorine atom; and $R_4$ and $R_6$ each represent a lower alkoxycarbonylamino group, etc.,
are disclosed in JP-B-46-519 (corresponding to Australian Patent 6,609,695A and Netherlands Patent 6,611,766A). They are different from the compounds of the present invention in kind of the substituents on the pyridine ring.

(4) Compounds represented by formula:

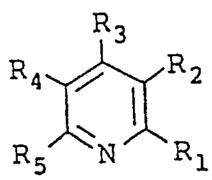

wherein $R_1$ and $R_3$ each represent a substituted amino group, e.g., an alkoxycarbonylamino group, a fluorine atom, a chlorine atom, etc.; and $R_2$, $R_4$, and $R_5$ each represent a fluorine atom or a chlorine atom; at least one of $R_1$, $R_2$, $R_3$, $R_4$, and $R_5$ representing a fluorine atom,
are disclosed as an effective ingredient of a tickicidal composition in South African Patent 710,118 and Australian Patent Publication 449,458. These compounds are different from the compounds of the present invention in kind of substituents on the pyridine ring.

(5) Compounds represented by formula:

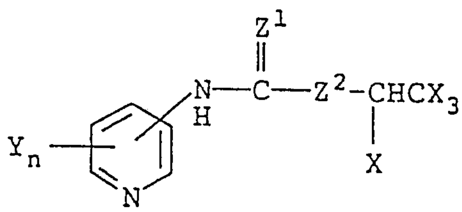

wherein X represents a halogen atom; $Z^1$ and $Z^2$ each represent an oxygen atom or a sulfur atom; Y represents a halogen atom or an alkyl group having from 1 to 6 carbon atoms; and n represents an integer of from 0 to 2,
are disclosed in U.S. Patent 3,547,934. They are different from the compounds of the present invention in combination of the substituents on the pyridine ring and the carbamic ester moiety.

(6) Compounds represented by formula:

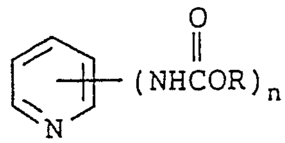

wherein R represents a lower alkenyl group or a phenyl group; n represents 1 or 2; and the para-position of the nitrogen hetero atom is unsubstituted,
are disclosed in U.S. Patent 3,364,225. They are different from the compounds of the present invention in kind of substituents on the pyridine ring.

(7) Compounds represented by formula:

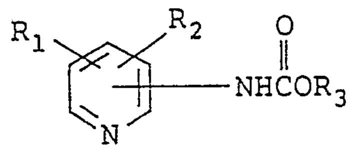

wherein $R_1$ represents a hydrogen atom, a phenyl group, or a lower alkyl group; $R_2$ represents a hydrogen atom, a phenyl group, or a lower alkyl group; and $R_3$ represents a pyridyl group, a piperidyl group, a pyridyl-lower alkyl group, a piperidyl-lower alkyl group, or an N-lower alkylpiperidyl group,
are disclosed in U.S. Patent 3,376,307. They are different from the compounds of the present invention in kind of the substituents on the pyridine ring and the carbamic ester moiety.

## SUMMARY OF THE INVENTION

An object of the present invention is to provide a novel pyridylcarbamate compound.

Another object of the present invention is to provide a process for preparing the pyridylcarbamate compound.

A further object of the present invention is to provide a biocidal composition containing the pyridylcarbamate compound for controlling harmful organisms.

The present invention relates to a pyridylcarbamate compound represented by formula (I):

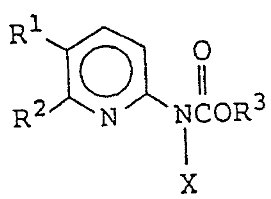

(I)

wherein $R^1$ represents a halogen atom, an alkoxy group, a trifluoromethyl group, a cyano group, or an alkylthio group; $R^2$ represents an alkyl group or an alkylthio group; $R^3$ represents a substituted or unsubstituted alkyl group, a substituted or unsubstituted alkenyl group, or a substituted or unsubstituted alkynyl group; and X represents a hydrogen atom, a substituted or unsubstituted alkyl group, or a substituted or unsubstituted acyl group; provided that a combination of $R^1$ representing a halogen atom, $R^2$ representing an alkyl group, $R^3$ representing an unsubstituted alkyl group or a tetrahalogenoethyl group, and X representing a hydrogen atom and a combination of $R^1$ representing an alkoxy group and $R^2$ representing an alkylthio group are excluded.

Of the compounds represented by formula (I), preferred are (i) those wherein $R^1$ is a halogen atom; $R^2$ is an alkyl group; $R^3$ is a substituted or unsubstituted alkynyl group; and X is a hydrogen atom; and (ii)

those wherein $R^1$ is an iodine atom; $R^2$ is an ethyl group; $R^3$ is a methyl group; and X is an ethyl group.

More preferred of the compounds (i) is a compound wherein $R^1$ is an iodine atom; $R^2$ is an ethyl group; $R^3$ is a propargyl group; and X is a hydrogen atom.

## DETAILED DESCRIPTION OF THE INVENTION

In formula (I), the alkyl group or alkyl moiety in $R^1$, $R^2$, $R^3$, and X includes those containing from 1 to 6 carbon atoms, e.g., methyl, ethyl, propyl, butyl, pentyl, and hexyl groups. The alkenyl group or alkenyl moiety in $R^3$ includes those containing from 3 to 6 carbon atoms, e.g., propenyl, butenyl, pentenyl, and hexenyl groups. The alkynyl group or alkynyl moiety in $R^3$ includes those containing from 3 to 6 carbon atoms, e.g., propynyl, butynyl, pentynyl, and hexynyl groups. Each of these groups or moieties embraces structural isomers having a straight or branched aliphatic chain.

The acyl group or acyl moiety in X includes an alkylcarbonyl group and a cycloalkylcarbonyl group, the cycloalkyl moiety of which includes those containing from 3 to 6 carbon atoms.

The halogen atom as represented by $R^1$ includes fluorine, chlorine, bromine, and iodine atoms.

Substituents of the substituted alkyl group, substituted alkenyl group and substituted alkynyl group as represented by $R^3$ and of the substituted alkyl group and substituted acyl group as represented by X include a halogen atom, an alkoxy group, and a cyano group. Where two or more substituents are bonded, they may be the same or different.

The compounds represented by formula (I) can be prepared by, for example, the following Reaction Steps 1 to 4:

Compound (I) Wherein X = H:

Step 1:

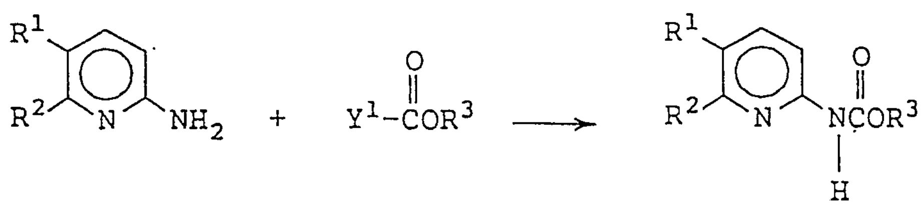

(II)

(I-a)

wherein $R^1$, $R^2$, and $R^3$ are as defined above; and $Y^1$ represents a halogen atom.

The reaction is usually carried out in a solvent, if desired, in the presence of a base. Suitable solvents include aromatic hydrocarbons, e.g., benzene, toluene, xylene, and chlorobenzene; cyclic or acyclic aliphatic hydrocarbons, e.g., chloroform, carbon tetrachloride, methylene chloride, dichloroethane, trichloroethane, n-hexane, and cyclohexane; ethers, e.g., diethyl ether, dioxane, and tetrahydrofuran; ketones, e.g., acetone, methyl ethyl ketone, and methyl isobutyl ketone; nitriles, e.g., acetonitrile and propionitrile; and aprotic polar solvents, e.g., dimethylformamide, N-methylpyrrolidone, dimethyl sulfoxide, and sulfolane. Bases which can be used may be either organic or inorganic. Examples of suitable inorganic bases include alkali metal hydroxides, e.g., sodium hydroxide and potassium hydroxide; alkali metal or alkaline earth metal carbonates, e.g., anhydrous potassium carbonate and anhydrous calcium carbonate; alkali metal hydrides, e.g., sodium hydride; and alkali metals, e.g., metallic sodium. Examples of suitable organic bases include triethylamine. The starting compound of formula (II) also serves as a base.

The reaction is performed at a temperature usually ranging from -30° to 100° C, and preferably from 0° to 60° C, for a period usually ranging from 1 to 24 hours, and preferably from 1 to 5 hours.

Step 2:

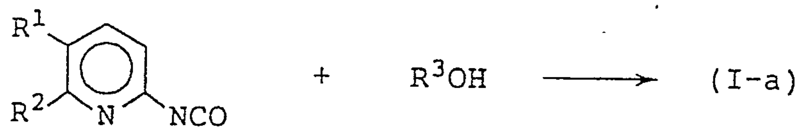

(III)

wherein $R^1$, $R^2$, and $R^3$ are as defined above.

The reaction is usually carried out in a solvent. Suitable solvents include aromatic hydrocarbons, e.g., benzene, toluene, xylene, and chlorobenzene, and cyclic or acyclic aliphatic hydrocarbons, e.g., chloroform, carbon tetrachloride, methylene chloride, dichloroethane, trichloroethane, n-hexane, and cyclohexane.

The reaction is performed at a temperature usually ranging from 0° to 100°C, and preferably from room temperature to 70°C, for a period usually ranging from 1 to 24 hours.

Compound (I) Wherein X = Substituted or Unsubstituted Alkyl Group or Substituted or Unsubstituted Acyl Group:

Step 3:

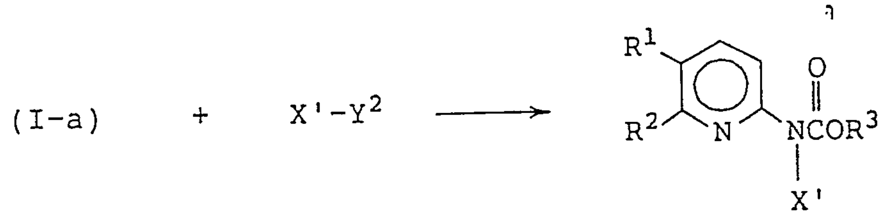

(I-b)

wherein $R^1$, $R^2$, and $R^3$ are as defined above; X' represents a substituted or unsubstituted alkyl group or a substituted or unsubstituted acyl group; and $Y^2$ represents a chlorine atom, a bromine atom, or an iodine atom.

The reaction is usually carried out in a solvent in the presence of a base. Solvents which can be used include those described in Step 1. Examples of suitable bases include alkali metal hydrides, e.g., sodium hydride, and alkali metal hydroxides, e.g., sodium hydroxide and potassium hydroxide. The reaction temperature is from 0° to 100°C, and the reaction completes within from 0.1 to 24 hours.

Compound (I) Wherein $R^3$ = $-(CH_2)_n\equiv CI$ (n: 1-4):

Step 4:

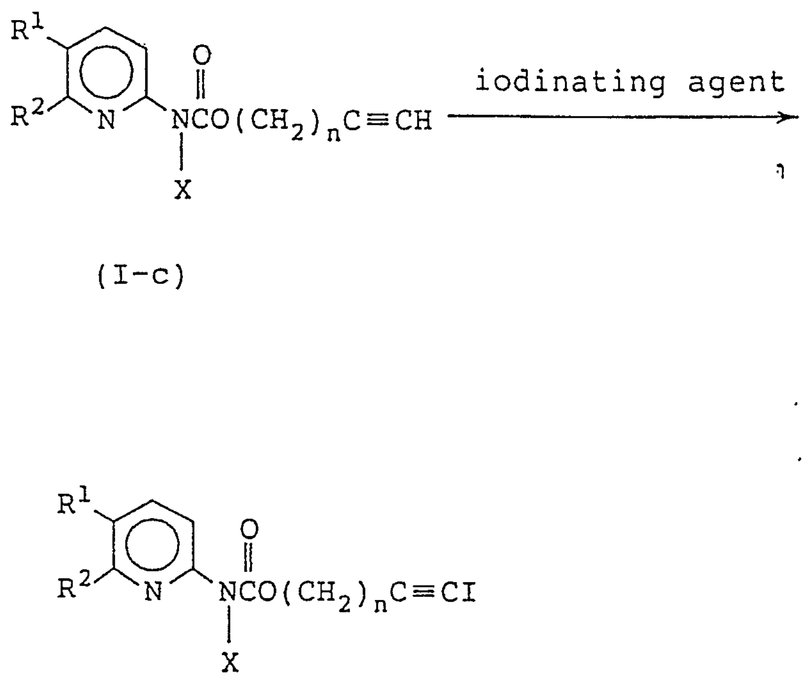


(I-c)

(I-d)

wherein $R^1$, $R^2$, X, and n are as defined above.

The iodinating agent to be used includes iodine. The reaction is usually carried out in a solvent, such as alcohols, e.g., methanol and ethanol, in the presence of an alkali, such as alkali metal hydroxides, e.g., sodium hydroxide and potassium hydroxide. The reaction temperature is from -10° C to room temperature, and the reaction completes within from 0.1 to 12 hours.

The starting compound represented by formula (II) can be synthesized by, for example, Reaction Steps 5 to 11 shown below.

(i) Compound (II) Wherein $R^1$ = Cℓ, Br or I:

Step 5:

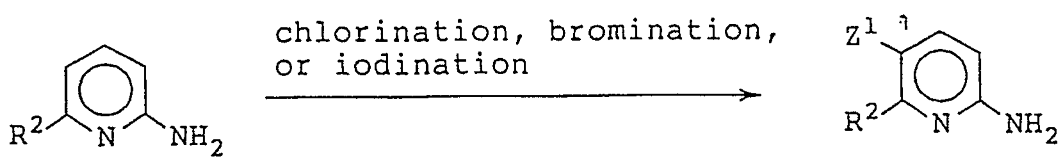


(IV)

(II-a)

wherein $R^2$ is as defined above; and $Z^1$ represents a chlorine atom, a bromine atom or an iodine atom.

The chlorination reaction is usually carried out by using chlorine, etc. as a chlorinating agent in the presence of sulfuric acid at a temperature of from -78° to 100° C, preferably from -50° to 30° C, for a period of from 0.1 to 24 hours.

The bromination reaction is usually carried out by using bromine, etc. as a brominating agent in the

presence of sulfuric acid at a temperature of from 0° to 100° C, preferably at room temperature, for a period of from 0.1 to 24 hours.

The iodination reaction is usually carried out by using iodine, etc. as an iodinating agent in the presence of an aqueous solution of potassium hydroxide or sodium hydroxide at a temperature of from 0° to 100° C, preferably at room temperature, for a period of from 0.1 to 24 hours.

(ii) Compound (II) Wherein $R^1$ = alkoxy:

Step 6:

(1)

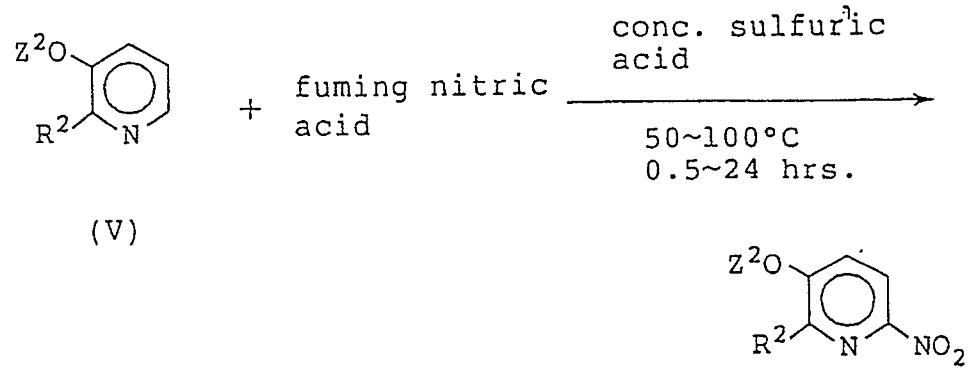


(V)

(VI-a)

(2)

(VI-a)

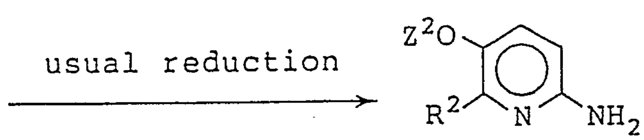


(II-b)

wherein $R^2$ is as defined above; and $Z^2$ represents an alkyl group.

The reduction reaction (Step 6-(2)) is carried out, for example, in an alcohol, e.g., ethanol, and/or water in the presence of stannous chloride and hydrochloric acid at a temperature of from 70° to 90° C for a period of from 2 to 5 hours.

(iii) Compound (II) Wherein $R^1$ = $CF_3$:

Step 7:

(1)

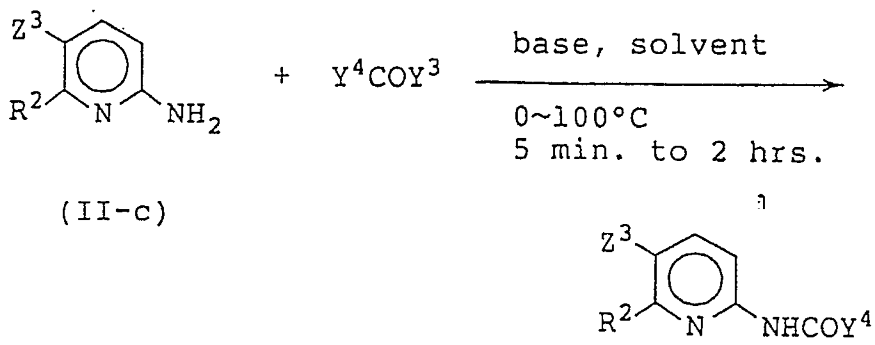


(II-c)

(VII-a)

(2) (VII-a) + $CF_3I$

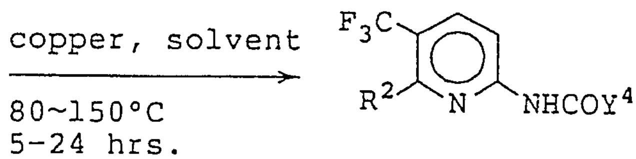


(VII-b)

(3) (VII-b)

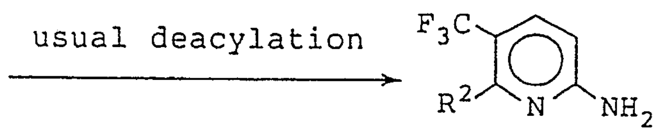


(II-d)

wherein $R^2$ is as defined above; $Z^3$ represents a bromine atom or an iodine atom; $Y^3$ represents a chlorine atom or a bromine atom; and $Y^4$ represents an alkyl group having from 1 to 4 carbon atoms.

Suitable bases which can be used in Step 7-(1) include alkali metal hydroxides, e.g., sodium hydroxide and potassium hydroxide, and alkali metal or alkaline earth metal carbonates, e.g., anhydrous potassium carbonate and anhydrous calcium carbonate.

Suitable solvents which can be used in Step 7-(1) include those enumerated in Step 1 above and, in addition, alcohols, e.g., ethanol.

Suitable solvents which can be used in Step 7-(2) include aprotic polar solvents, e.g., dimethylformamide and dimethyl sulfoxide, and pyridine.

The deacylation reaction in Step 7-(3) is carried out, for example, in water and/or an alcohol in the presence of a base, e.g., sodium hydroxide and potassium hydroxide, at a temperature of from 40 to 100° C for a period of from 0.5 to 24 hours.

<u>(iv) Compound (II) Wherein $R^1 = CF_3$, $R^2 = C_2H_5$:</u>

<u>Step 8:</u>

(1)

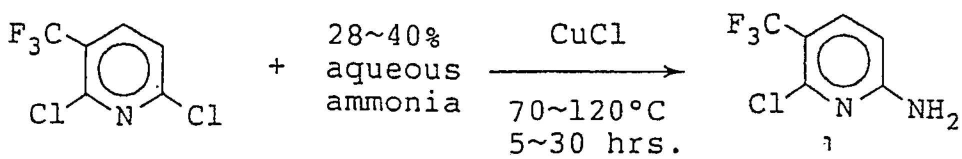


(VIII-a)

(2)

(VIII-a) + hydrogen bromide

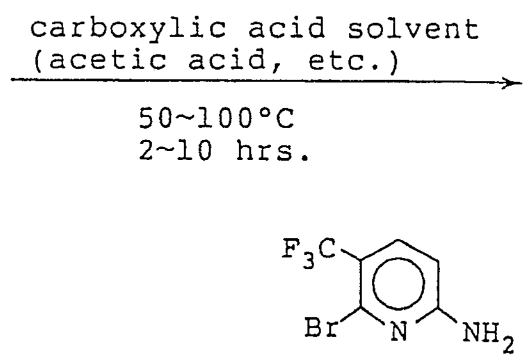


(VIII-b)

(3)

(VIII-b) + $Y^4COY^3$

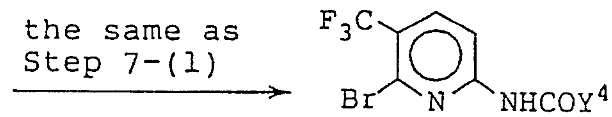


(VIII-c)

(4)

(VIII-c)

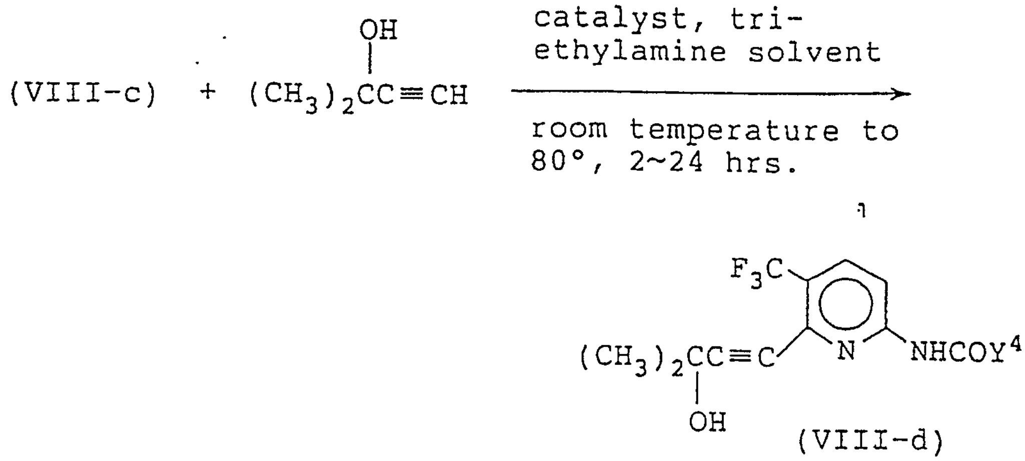


(VIII-d)

(5)

(VIII-d)

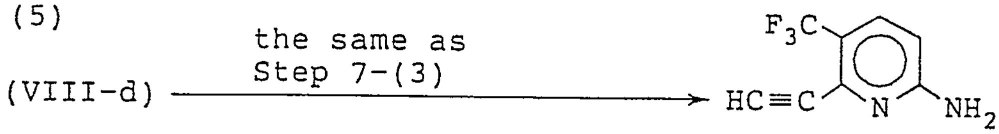


(VIII-e)

(6)

(VIII-e)

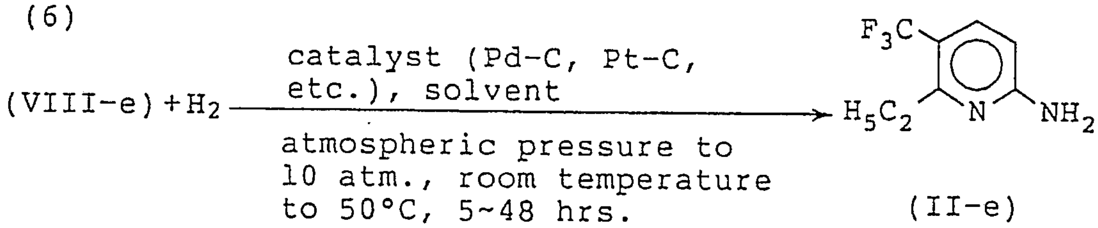


(II-e)

wherein $Y^3$ and $Y^4$ are as defined above.

Examples of the catalyst which can be used in Step 8-(4) include a mixture of CuI with palladium acetate or with bis(triphenylphosphine)palladium (II) chloride and, if desired, triphenylphosphine.

Suitable solvents which can be used in Step 8-(6) include alcohols, e.g., methanol and ethanol, and ethers, e.g., dioxane and tetrahydrofuran.

(v) Compound (II) Wherein $R^1$ = CN:

Step 9:

(1) (IV) 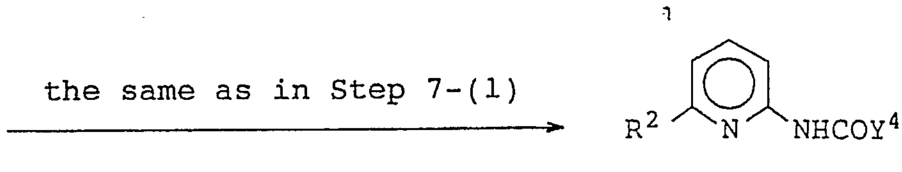


(IX-a)

(2) (IX-a) 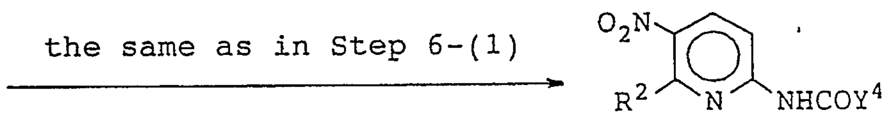


(IX-b)

(3) (IX-b) 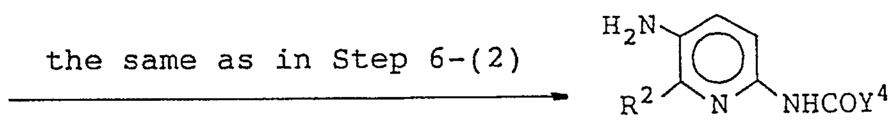


(IX-c)

(4) (IX-c) 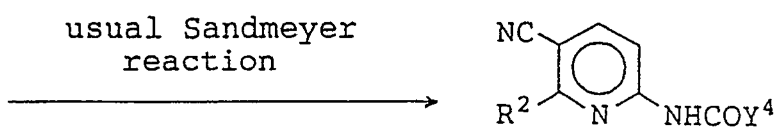


(IX-d)

(5) (IX-d) 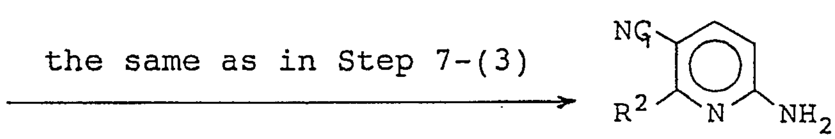


(II-f)

wherein $R^2$ and $Y^4$ are as defined above.

The Sandmeyer reaction in Step 9-(4) can be carried out by, for example, dissolving the compound (IX-

c) in concentrated hydrochloric acid, adding dropwise an aqueous solution of sodium nitrite to the solution at 0° to 5° C to form a diazonium salt, neutralizing the mixture with an aqueous solution of sodium bicarbonate, adding cuprous cyanide, and gradually returning the mixture to room temperature.

(vi) Compound (II) Wherein $R^1$ = $SZ^2$:

Step 10:

(1) (II-a)

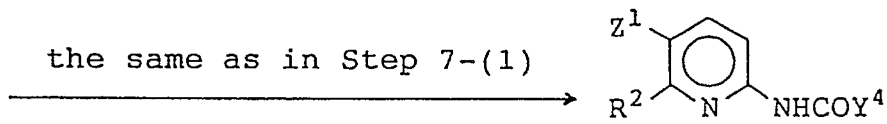


(X-a)

(2) (X-a) + $(Z^2S)_2$

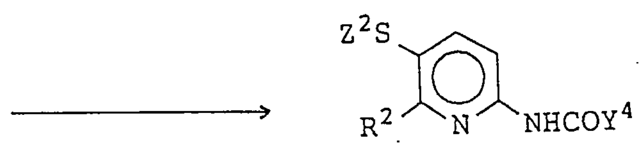

(X-b)

(3) (X-b)

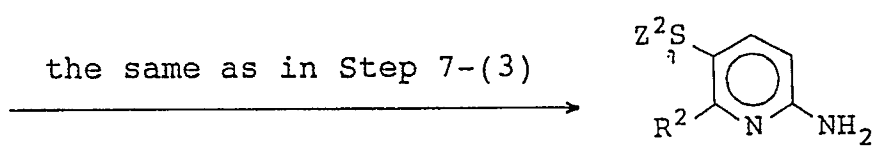


(II-g)

wherein $R^2$, $Y^4$, $Z^1$, and $Z^2$ are as defined above.

Step 10-(2) can be carried out by dissolving the compound (X-a) in an ether, e.g., diethyl ether and tetrahydrofuran, adding an alkali metal hydride, e.g., sodium hydride, thereto to conduct reaction at 0° to 50° C for 10 minutes to 1 hour, followed by cooling to -100° to -50° C, reacting with butyl lithium, adding $(Z^2S)_2$ (wherein $Z^2$ is as defined above) thereto, and gradually returning the mixture to room temperature.

(vii) Compound (II) Wherein $R^1$ = F:

Step 11:

The titled compound can be synthesized, for example, in accordance with the process described in Journal of Heterocyclic Chemistry, Vol. 24, pp. 215-217 (1987) by starting with the compound (IX-c) obtained in Step 9-(3).

The compound represented by formula (IV) used in Step 5 can be prepared, for example, in accordance with Reaction Steps 12 and 13 shown below.

(i) Compound (IV) Wherein $R^2$ = alkyl:

Step 12:

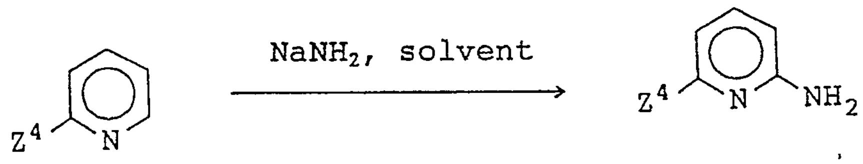


(IV-a)

wherein $Z^4$ represents an alkyl group.

Examples of solvents which can be used in Step 12 include aromatic compounds, e.g., toluene, xylene, N,N-dimethylaniline, and N,N-diethylaniline. The reaction is usually carried out at a temperature of from 100° to 200° C, preferably from 120° to 150° C, for a period of from 1 to 60 hours. The amount of $NaNH_2$ to be used ranges from 1 to 2 moles per mol of the starting compound.

(ii) Compound (IV) Wherein $R^2$ = alkylthio:

Step 13:

(1)

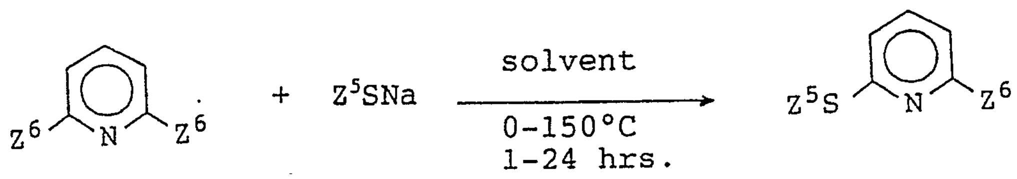


(XI-a)

(2) (XI-a)

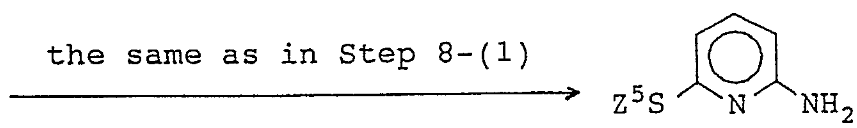


(IV-b)

wherein $Z^5$ represents an alkyl group; and $Z^6$ represents a chlorine atom or a bromine atom.

The solvent which can be used in Step 13-(1) includes ethers, e.g., dioxane and tetrahydrofuran, and aprotic polar solvents, e.g., dimethyl sulfoxide and dimethylformamide.

The compounds represented by formula (V) to be used in Step 6 can be prepared, for example, by Reaction Steps 14 and 15 shown below.

(i) Compound (V) Wherein $R^2$ = $CH_3$:

Step 14:

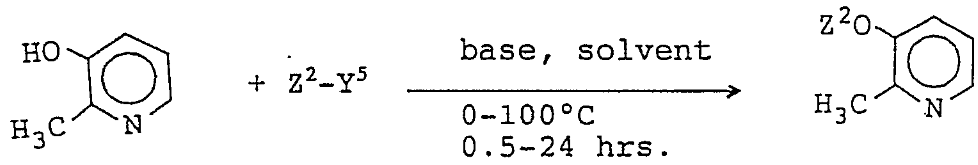


(V-a)

wherein $Z^2$ is as defined above; and $Y^5$ represents a chlorine atom, a bromine atom, or an iodine atom.

Examples of the base and solvent which can be used in Step 14 are the same as those enumerated for Step 1.

(ii) Compound (V) Wherein $R^2$ = $C_{2-6}$ alkyl:

Step 15:

(1)

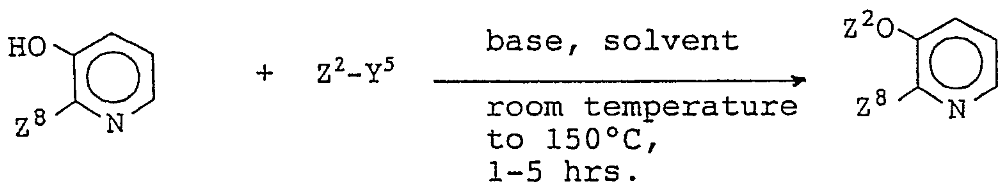


(XII-a)

(2)

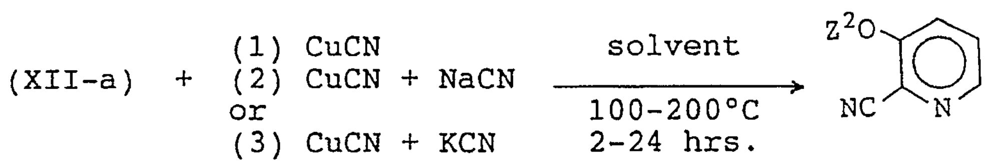


(XII-b)

(3) The compound (XII-b) and $Y^5$-Mg-$Z^7$ are reacted in a nitrogen atmosphere in the presence of an ether solvent at 0° to 50°C for 0.5 to 5 hours. After cooling the reaction mixture, concentrated hydrochloric acid is added thereto to conduct reaction at 40° to 80°C for 2 to 10 hours to obtain a compound of formula (XII-c):

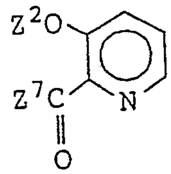

(XII-c)

(4) (XII-c) + $H_2NNH_2$

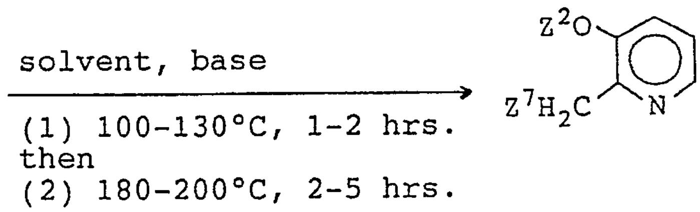


(V-b)

wherein $Z^2$ is as defined above; $Y^5$ and $Z^8$ each represent a chlorine atom, a bromine atom, or an iodine atom; and $Z^7$ represents an alkyl group having from 1 to 5 carbon atoms.

Suitable solvents which can be used in Steps 15-(1) and 15-(2) include aprotic solvents, e.g., dimethylformamide, dimethyl sulfoxide, and sulfolane. Suitable bases which can be used in Step 15-(1) include alkali metal or alkaline earth metal carbonates, e.g., anhydrous sodium carbonate and anhydrous potassium carbonate, and alkali metal hydroxides, e.g., sodium hydroxide and potassium hydroxide. Examples of solvents which can be used in Step 15-(4) include ethylene glycols, e.g., ethylene glycol and triethylene glycol. Bases which can be used in Step 15-(4) include alkali metal hydroxides, e.g., sodium hydroxide and potassium hydroxide.

The compounds represented by formula (III) which can be used in Step 2 can be prepared, for example, according to Reaction Step 16 shown below.

Step 16:

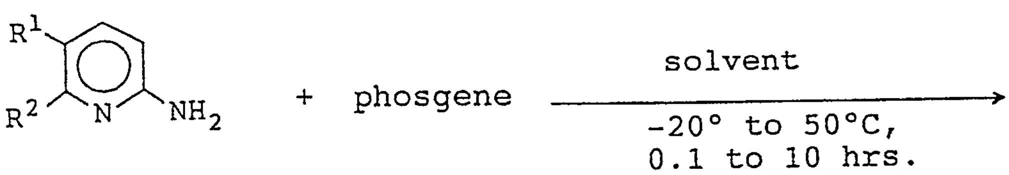


(III)

(II)

wherein $R^1$ and $R^2$ are as defined above.

Examples of solvents which can be used in Step 16 include organic solvents, e.g., benzene, toluene, xylene, and ethyl acetate.

Examples of synthesis of the compounds represented by formula (I) according to the present invention are described below for illustrative purposes only but not for limitation.

SYNTHESIS EXAMPLE 1

Synthesis of Propargyl N-(6-Ethyl-5-iodo-2-pyridyl)carbamate (Compound No. 1)

In 10 mℓ of anhydrous diethyl ether was dissolved 1.55 g of propargyl chloroformate obtained by reacting propargyl alcohol and phosgene. To the solution was added 3.24 g of 2-amino-6-ethyl-5-iodopyridine under ice cooling, followed by stirring at room temperature for 5 hours. The reaction mixture was washed with diluted hydrochloric acid and dried over anhydrous sodium sulfate. The solvent was

removed by distillation under reduced pressure, and the residue was purified by column chromatography using methylene chloride as a developing solvent to obtain 1.64 g of the titled compound (Compound No. 1) having a melting point of 78 to 79 °C.

SYNTHESIS EXAMPLE 2

Synthesis of 3-Iodopropargyl N-(6-Ethyl-5-iodo-2-pyridyl)carbamate (Compound No. 2)

In 20 mℓ of a methanol solution containing 0.156 g of sodium hydroxide was dissolved 1.3 g of propargyl N-(6-ethyl-5-iodo-2-pyridyl)carbamate (Compound No. 1) obtained in Synthesis Example 1. To the solution was added 1 g of iodine under ice cooling, followed by stirring at room temperature for 2 hours. The reaction mixture was poured into water, and the precipitated crystals were filtered and dried to obtain 0.2 g of the titled compound (Compound No. 2) having a melting point of 124-126 °C.

SYNTHESIS EXAMPLE 3

Synthesis of Methyl N-Ethyl-N-(6-ethyl-5-iodo-2-pyridyl)carbamate (Compound No. 50)

In 10 mℓ of dimethylformamide was dissolved 1.0 g of methyl N-(6-ethyl-5-iodo-2-pyridyl)carbamate, and 0.16 g of sodium hydride was added thereto, followed by stirring at room temperature for 20 minutes. Then, 0.51 g of ethyl iodide was added to the reaction mixture, followed by stirring at room temperature over night. Water was added to the reaction mixture, and the mixture was extracted with methylene chloride. The extract was dried over anhydrous sodium sulfate, and methylene chloride was removed therefrom by distillation under reduced pressure. The residue was purified by column chromatography using ethyl acetate/hexane (1/4 by volume) as a developing solvent to obtain the titled compound (Compound No. 50) having a melting point of 48 to 50 °C.

Specific examples of the intermediate compounds represented by formula (II) are shown in Table 1 below.

TABLE 1

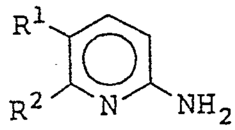

(II)

| Compound No. | $R^1$ | $R^2$ | Physical Properties |
|---|---|---|---|
| II-1 | -I | $-CH_2CH_3$ | m.p. 62-65°C |
| II-2 | -I | $-CH_2CH(CH_3)_2$ | - |
| II-3 | $-SCH_3$ | $-SCH_3$ | - |
| II-4 | -I | $-CH_2CH_2CH_3$ | - |
| II-5 | -I | $-(CH_2)_3CH_3$ | - |
| II-6 | -Cl | $-CH_2CH_3$ | - |
| II-7 | -Br | " | - |
| II-8 | $-CF_3$ | " | m.p. 66-70°C |
| II-9 | -CN | " | - |
| II-10 | $-OCH_3$ | " | - |
| II-11 | $-OC_2H_5$ | " | oily |
| II-12 | -I | $-SCH_3$ | - |
| II-13 | $-CF_3$ | $-SC_2H_5$ | - |
| II-14 | -CN | $-SCH_3$ | - |
| II-15 | -CN | $-SC_2H_5$ | - |
| II-16 | $-SCH_3$ | $-CH_2CH_3$ | - |
| II-17 | $-SC_2H_5$ | $-SC_2H_5$ | - |
| II-18 | -I | " | - |

Specific examples of the compounds (I) according to the present invention are shown in Tables 2 and 3 below.

TABLE 2

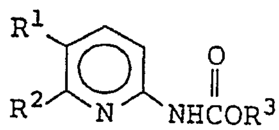

(I-a)

| Compound No. | $R^1$ | $R^2$ | $R^3$ | Physical Properties |
|---|---|---|---|---|
| 1 | -I | $-CH_2CH_3$ | $-CH_2C\equiv CH$ | m.p. 78-79°C |
| 2 | " | " | $-CH_2C\equiv CI$ | m.p. 124-126°C |
| 3 | " | " | $-CH_2CH_2C\equiv CH$ | m.p. 92-95°C |
| 4 | " | " | $-CH_2C\equiv CCH_3$ | m.p. 90-93°C |
| 5 | " | " | $CH_3$<br>\|<br>$-CH-C\equiv CH$ | m.p. 54-55°C |
| 6 | -Br | $-CH_3$ | $-CH_2C\equiv CH$ | - |
| 7 | -I | " | " | m.p. 101-103°C |
| 8 | " | $-CH_2CH_2CH_3$ | " | m.p. 68-70°C |
| 9 | -Cl | $-CH_2CH_3$ | " | m.p. 70-72°C |
| 10 | -Br | " | " | m.p. 84-86°C |
| 11 | -I | " | $-CH_2C\equiv CBr$ | - |
| 12 | " | " | $-CH_2C\equiv CCl$ | - |
| 13 | " | " | $-CH_2CH_2Cl$ | m.p. 54-60°C |
| 14 | " | " | $-CH_2CH_2CN$ | m.p. 108-110°C |

/To be cont'd.

TABLE 2 (Cont'd.)

| Compound No. | $R^1$ | $R^2$ | $R^3$ | Physical Properties |
|---|---|---|---|---|
| 15 | -I | $-CH_2CH_3$ | $-CH_2CH_2OC_2H_5$ | m.p. 36-38°C |
| 16 | " | " | $-CH_2CH=CH_2$ | m.p. 42-43°C |
| 17 | " | " | 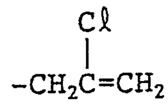 | refractive index: $n_D^{26.1}$ 1.5964 |
| 18 | " | " | $-CH_2C{\equiv}CCH_2Br$ | m.p. 110-114°C |
| 19 | -CN | " | $-CH_3$ | m.p. 130-137°C |
| 20 | $-CF_3$ | " | $-CH_2C{\equiv}CH$ | refractive index: $n_D^{26.1}$ 1.4586 |
| 21 | $-OCH_3$ | " | " | m.p. 123-125°C |
| 22 | $-OC_2H_5$ | " | " | m.p. 102-104°C |
| 23 | $-OC_3H_7(n)$ | " | " | m.p. 92-94°C |
| 24 | -I | $-SCH_3$ | " | m.p. 120-124°C |
| 25 | " | $-(CH_2)_3CH_3$ | " | m.p. 61-62°C |
| 26 | " | $-CH(CH_3)_2$ | " | m.p. 74-78°C |
| 27 | " | $-SC_2H_5$ | " | - |
| 28 | $-OC_2H_5$ | $-CH_3$ | " | - |
| 29 | -F | $-CH_2CH_3$ | " | - |
| 30 | $-CF_3$ | " | $-CH_2CH_2Cl$ | - |
| 31 | $-OC_2H_5$ | " | " | - |
| 32 | $-CF_3$ | $-CH_2CH_2CH_3$ | $-CH_2C{\equiv}CH$ | - |
| 33 | $-OCH_3$ | $-CH_2CH_3$ | $-CH_3$ | - |

/To be cont'd.

TABLE 2 (Cont'd.)

| Compound No. | $R^1$ | $R^2$ | $R^3$ | Physical Properties |
|---|---|---|---|---|
| 34 | $-OC_2H_5$ | $-CH_2CH_3$ | $-CH_3$ | - |
| 35 | $-OC_3H_7$(n) | " | " | - |
| 36 | $-OCH_3$ | " | $-CH_2CH_2C{\equiv}CH$ | - |
| 37 | $-OC_2H_5$ | " | " | - |
| 38 | $-OC_3H_7$(n) | " | " | - |
| 39 | -Cl | " | " | - |
| 40 | -Br | " | " | - |
| 41 | $-SCH_3$ | " | $-CH_3$ | m.p. 75-76°C |
| 42 | " | $-SCH_3$ | " | - |
| 43 | " | " | $-CH_2C{\equiv}CH$ | - |
| 44 | $-SC_2H_5$ | $-SC_2H_5$ | $-CH_3$ | - |
| 45 | " | " | $-CH_2C{\equiv}CH$ | - |
| 46 | -I | $-CH_2CH_3$ | 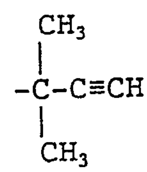 | m.p. 44-47°C |
| 47 | " | " | 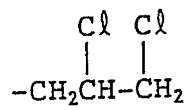 | refractive index: $n_D^{14.4}$ 1.6024 |
| 48 | " | " | $-CH_2C{\equiv}CC_2H_5$ | m.p. 87-90°C |

TABLE 3

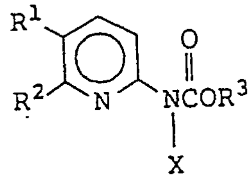

(I-b)

| Compound No. | $R^1$ | $R^2$ | X | $R^3$ | Physical Properties |
|---|---|---|---|---|---|
| 49 | -I | $-CH_2CH_3$ | $-CH_3$ | $-CH_3$ | m.p. 28-30°C |
| 50 | " | " | $-CH_2CH_3$ | " | m.p. 48-50°C |
| 51 | -Br | $-CH_3$ | $-CH_2CH_2CH_3$ | " | - |
| 52 | -I | $-CH_2CH_3$ | $-COCH_3$ | " | m.p. 55-57°C |
| 53 | " | " | -CO- 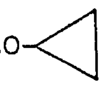 | " | m.p. 91-94°C |
| 54 | " | " | $-CH_2CH_3$ | $-CH_2C{\equiv}CH$ | m.p. 55-57°C |
| 55 | " | $-CH_3$ | " | $-CH_3$ | m.p. 33-36°C |
| 56 | " | $-CH_2CH_2CH_3$ | " | " | m.p. 51-53°C |
| 57 | " | $-CH_2CH_3$ | " | $-CH_2CH_2CH_3$ | oily |
| 58 | -Cℓ | " | $-CH_3$ | $-CH_3$ | - |
| 59 | -I | $-SCH_3$ | $-CH_2CH_3$ | " | - |
| 60 | $-CF_3$ | $-CH_2CH_3$ | " | " | - |
| 61 | $-OCH_3$ | " | " | " | - |
| 62 | " | " | -CO- 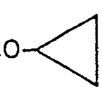 | " | - |

/To be cont'd.

TABLE 3 (Cont'd.)

| Compound No. | $R^1$ | $R^2$ | X | $R^3$ | Physical Properties |
|---|---|---|---|---|---|
| 63 | $-OC_2H_5$ | $-CH_2CH_3$ | $-CH_2CH_3$ | $-CH_3$ | - |
| 64 | " | " | -CO- 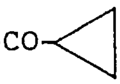 | " | - |
| 65 | $-OC_3H_7(n)$ | " | $-CH_2CH_3$ | " | - |
| 66 | " | " | -CO- 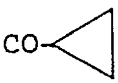 | " | - |
| 67 | -I | " | $-CH(CH_3)_2$ | " | - |
| 68 | " | " | $-COCH_2CH_3$ | " | - |
| 69 | " | " | $-COCH_2CH_2CH_3$ | " | - |
| 70 | " | " | $-COCH(CH_3)_2$ | " | - |
| 71 | -Br | " | -CO- 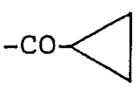 | " | - |
| 72 | " | " | $-CH_2CH_3$ | " | - |
| 73 | -Cl | " | -CO- 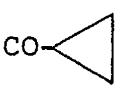 | " | - |
| 74 | " | " | $-CH_2CH_3$ | " | - |

/To be cont'd.

TABLE 3 (Cont'd.)

| Compound No. | $R^1$ | $R^2$ | X | $R^3$ | Physical Properties |
|---|---|---|---|---|---|
| 75 | -I | $-CH_2CH_3$ | -CO- 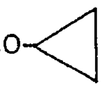 | $-CH_3$ | - |
| 76 | " | " | $-CH_2CH_3$ | " | - |
| 77 | " | " | $-CH_2OCH_3$ | " | refractive index: $n_D^{23.5}$ 1.5658 |

The compounds represented by formula (I) according to the present invention are useful as an active ingredient of biocidal compositions for controlling harmful organisms, such as agricultural and horticultural fungicides, medical antimicrobial agents, industrial bactericides, and agricultural and horticultural insecticides. For example, they exhibit excellent effects on controlling plant diseases, such as rice blast, rice sheath blight, cucumber anthracnose, cucumber powdery mildew, cucumber downy mildew, tomato late blight, tomato early blight, citrus melanose, citrus common green mold, apple and pear scab, apple alternaria blotch, grape downy mildew, and gray mold, sclerotinia rot and rust of various crops; and soil diseases caused by phytopathogenic fungi, e.g., Fusarium, Pythium, Rhizoctonia, Verticillium, and Plsmodiophora. In particular, the compounds exhibit excellent controlling effects on cucumber anthracnose, cucumber powdery mildew, citrus common green mold, apple and pear scab, and gray mold and sclerotinia rot of various crops. Besides excellent in preventive effects, the compounds of the present invention have excellent curative effects so that they are useful in the treatment after infection. Moreover, foliar diseases can be controlled by soil treatment. The compounds are effective on not only sensitive strains but various resistant strains, e.g., Benomyl-resistant Sphaerotheca fuliginea, Benomyl-resistant Botrytis cinerea, and dicarboximide-resistant Botrytis cinerea.

In addition, the compounds of the present invention also exhibit excellent controlling effects on agriculturally or horticulturally harmful insects, such as planthoppers, diamondback moth (Plutella xylostella), green rice leafhopper (Nephotettix cincticeps), adzuki bean weevil (Callosobruchus chinensis), common cutworm (Spodoptera litura), and green peach aphid (Myzus persicae), etc.; mites, e.g., two-spotted spider mite (Tetranychus urticae), carmine spider mite (Tetranychus cinnabarinus), and citrus red mite (Panonychus citri); and nematodes, e.g., southern root-knot nematode (Meloidogyne incognita).

The compounds of the present invention can be formulated into a variety of preparation forms of biocidal compositions, such as emulsifiable concentrates, dusts, wettable powders, aqueous solutions, granules, and suspensions (e.g., aqueous suspensions), together with adjuvants, as in conventional formulations. On actual use of these preparations, they may be used as such or as diluted with a diluent such as water to a predetermined concentration.

Adjuvants which can be used include carriers, emulsifying agents, suspending agents, dispersing agents, spreaders, penetrating agents, wetting agents, thickeners, and stabilizers. These adjuvants are added in proper amounts, if desired.

Carriers are classified into solid carriers and liquid carriers. Solid carriers include animal and vegetable powders, e.g., starch, sugar, cellulose powders, cyclodextrin, activated carbon, soybean powders, wheat powders, chaff powders, wood powders, fish powders, and powdery milk; and mineral powders, e.g., talc, kaolin, bentonite, lipophilic bentonite, calcium carbonate, calcium sulfate, sodium bicarbonate, zeolite, diatomaceous earth, white carbon, clay, alumina, silica, and sulfur powders. Liquid carriers include water; animal and vegetable oils, e.g., soybean oil and cotton seed oil; alcohols, e.g., ethyl alcohol and ethylene glycol; ketones, e.g., acetone and methyl ethyl ketone; ethers, e.g., dioxane and tetrahydrofuran; aliphatic hydrocarbons, e.g., kerosene, lamp oil, and liquid paraffin; aromatic hydrocarbons, e.g., xylene, trimethylbenzene, tetramethylbenzene, cyclohexane, and solvent naphtha; halogenated hydrocarbons, e.g., chloroform and chlorobenzene; acid amides, e.g., dimethylformamide; esters, e.g., ethyl acetate and fatty acid glycerin esters; nitriles, e.g., acetonitrile; sulfur-containing compounds, e.g., dimethyl sulfoxide; and N-methylpyrrolidone.

In addition to the above-described carriers, adjuvants include surface active agents serving as emulsifying agents, suspending agents, dispersing agents, spreaders, penetrating agents, wetting agents, thickeners, or stabilizers. Specific examples of suitable surface active agents are polyoxyethylene alkylaryl ethers, polyoxyethylene glycol nonylphenyl ether, polyoxyethylene lauryl ether, polyoxyethylene hardened caster oil, polyoxyethylene alkylaryl sulfates (e.g., polyoxyethylene alkylphenyl ether sulfates), polyoxyethylene fatty acid esters (e.g., polyoxyethylene stearate), polyoxyethylene sorbitan fatty acid esters, lower alcohol phosphates, sodium alkylsulfates, sodium lignin sulfonate, calcium lignin sulfonate, alkylaryl sulfonates, sodium alkylbenzenesulfonates, sodium $\beta$-naphthalene sulfonate-formalin condensates, dialkyl sulfosuccinates, oxyethylated polyarylphenol phosphates, and sodium methylnaphthalene sulfonate condensate.

A suitable mixing ratio of the compound of the present invention to these adjuvants is usually from 0.05:99.95 to 90:10, and preferably from 0.2:99.8 to 80:20, by weight.

A concentration of the compound to be applied varies depending on the crop to be applied, the method of application, the preparation form, the dose, and the like. In general, it is from 0.1 to 10,000 ppm, and preferably from 1 to 2,000 ppm, in the case of foliar treatment; and from 10 to 100,000 g/ha, and preferably from 200 to 20,000 g/ha, in the case of soil treatment.

If desired, the compound of the present invention may be used in combination with other agricultural chemicals, such as insecticides, miticides, nematocides, fungicides, antiviral agents, attractants, herbicides, and plant growth regulators. Such a combined use brings about further enhanced effects in some cases. Specific examples of the agricultural chemicals which can be used in combination are shown below.

Pyrimidinamine Compounds:

| Compound No. | Compound Name | Common Name |
|---|---|---|
| A-1 | 2-Anilino-4-methyl-6-(1-propynyl)pyrimidine | disclosed in JP-A-63-208581 |

Azole Compounds:

| Compound No. | Compound Name | Common Name |
|---|---|---|
| B-1 | 1-(4-Chlorophenoxy)-3,3-dimethyl-1-(1H-1,2,4-triazol-1-yl)butanone | Triadimefon |
| B-2 | 1-(Biphenyl-4-yloxy)-3,3-dimethyl-1-(1H-1,2,4-triazol-1-yl)butan-2-ol | Bitertanol |
| B-3 | 1-[N-(4-Chloro-2-trifluoromethylphenyl)-2-propoxy-acetimidoyl]imidazole | Triflumizole |
| B-4 | 1-[2-(2,4-Dichlorophenyl)-4-ethyl-1,3-dioxolan-2-yl-methyl]-1H-1,2,4-triazole | Etaconazole |
| B-5 | 1-[2-(2,4-Dichlorophenyl)-4-propyl-1,3-dioxolan-2-yl-methyl]-1H-1,2,4-triazole | Propiconazole |
| B-6 | 1-[2-(2,4-Dichlorophenyl)pentyl]-1H-1,2,4-triazole | Penconazole |
| B-7 | Bis(4-fluorophenyl)(methyl)(1H-1,2,4-triazol-1-yl-methyl)silane | Flusilazole |

| | | |
|---|---|---|
| B-8 | 2-(4-Chlorophenyl)-2-(1H-1,2,4-triazol-1-ylmethyl)-hexanenitrile | Myclobutanil |
| B-9 | (2RS,3RS)-2-(4-Chlorophenyl)-3-cyclopropyl-1-(1H-1,2,4-triazol-1-yl)butan-2-ol | Cyproconazole |
| B-10 | (RS)-1-(4-Chlorophenyl)-4,4-dimethyl-3-(1H-1,2,4-triazol-1-ylmethyl)pentan-3-ol | Terbuconazole |
| B-11 | (RS)-2-(2,4-Dichlorophenyl)-1-(1H-1,2,4-triazol-1-yl)hexan-2-ol | Hexaconazole |
| B-12 | (2RS,5RS)-5-(2,4-Dichlorophenyl)tetrahydro-5-(1H-1,2,4-triazol-1-ylmethyl)-2-furyl 2,2,2-trifluoro-ethyl ether | Furconazole-cis |
| B-13 | N-Propyl-N-[2-(2,4,6-trichlorophenoxy)ethyl]-imidazole-1-carboxamide | Prochloraz |

Quinoxaline Compounds:

| Compound No. | Compound Name | Common Name |
|---|---|---|
| C-1 | 6-Methyl-1,3-dithiolo[4,5-b]quinoxalin-2-one | Chinomethionate |

Dithiocarbamate Compounds:

| Compound No. | Compound Name | Common Name |
|---|---|---|
| D-1 | Manganese ethylenebis(dithiocarbamate) polymer | Maneb |
| D-2 | Zinc ethylenebis(dithiocarbamate) polymer | Zineb |

| | | |
|---|---|---|
| D-3 | Complex of zinc and manganese ethylenebis(dithiocarbamate) (Maneb) | Mancozeb |
| D-4 | Dizinc bis(dimethyldithiocarbamate) ethylenebis-(dithiocarbamate) | Polycarbamate |
| D-5 | Zinc propylenebis(dithiocarbamate) polymer | Propineb |

Organic Chlorine Compounds:

| Compound No. | Compound Name | Common Name |
|---|---|---|
| E-1 | 4,5,6,7-Tetrachlorophthalide | Fthalide |
| E-2 | Tetrachloroisophthalonitrile | Chlorothalonil |
| E-3 | Pentachloronitrobenzene | Quintozene |
| E-4 | 2,2,2-Trichloro-1,1-bis(4-chlorophenyl)ethanol | Dicofol |

Benzimidazole Compounds:

| Compound No. | Compound Name | Common Name |
|---|---|---|
| F-1 | Methyl 1-(butylcarbamoyl)benzimidazol-2-yl carbamate | Benomyl |
| F-2 | Dimethyl 4,4'-(o-phenylene)-bis(3-thioallophanate) | Thiophanate-Methyl |
| F-3 | Methyl benzimidazol-2-ylcarbamate | Carbendazim |

Pyridinamine Compounds:

| Compound No. | Compound Name | Common Name |
|---|---|---|
| G-1 | 3-Chloro-N-(3-chloro-2,6-dinitro-4-α,α,α-trifluoro-tolyl)-5-trifluoromethyl-2-pyridinamine | Fluazinam |

Cyanoacetamide Compounds:

| Compound No. | Compound Name | Common Name |
|---|---|---|
| H-1 | 1-(2-Cyano-2-methoxyiminoacetyl)-3-ethylurea | Cymoxanil |

Phenylamide Compounds:

| Compound No. | Compound Name | Common Name |
|---|---|---|
| I-1 | Methyl N-(2-methoxyacetyl)-N-(2,6-xylyl)-DL-alaninate | Metalaxyl |
| I-2 | 2-Methoxy-N-(2-oxo-1,3-oxazolidin-3-yl)acet-2',6'-xylidide | Oxadixyl |
| I-3 | (±)-α-2-Chloro-N-(2,6-xylylacetamide)-γ-butyrolactone | Ofurace |
| I-4 | Methyl N-phenylacetyl-N-(2,6-xylyl)-DL-alaninate | Benalaxyl |
| I-5 | Methyl N-(2-furoyl)-N-(2,6-xylyl)-DL-alaninate | Furalaxyl |
| I-6 | (±)-α-[N-(3-Chlorophenyl)cyclopropanecarboxamide]-γ-butyrolactone | Cyprofuram |

Sulfenic Acid Compounds:

| Compound No. | Compound Name | Common Name |
| --- | --- | --- |
| J-1 | N-Dichlorofluoromethylthio-N',N'-dimethyl-N-phenyl-sulfamide | Dichlofluanid |

Copper Compounds:

| Compound No. | Compound Name | Common Name |
| --- | --- | --- |
| K-1 | Copper hydroxide | Copper Hydroxide |
| K-2 | Copper 8-quinolinolate | Oxine-Copper |

Isoxazole Compounds:

| Compound No. | Compound Name | Common Name |
| --- | --- | --- |
| L-1 | 5-Methylisoxazol-3-ol | Hydroxyisoxazole |

Organophosphorus Compounds:

| Compound No. | Compound Name | Common Name |
| --- | --- | --- |
| M-1 | Aluminum tris(ethyl phosphonate) | Fosetyl-Al |
| M-2 | O-2,6-Dichloro-p-tolyl-O,O-dimethyl phosphorothioate | Tolclofosmethyl |

| | | |
|---|---|---|
| M-3 | (RS)-S-(RS)-sec-Butyl-O-ethyl-2-oxo-2-thiazolidinyl-phosphonothioate | disclosed in U.S. Patent 4,590,182 |
| M-4 | O,S-Dimethyl acetylphosphoramidothioate | Acephate |
| M-5 | 2,2-Dichlorovinyl dimethylphosphate | Dichlorvos |
| M-6 | O-2,4-Dichlorophenyl O-ethyl S-propyl phosphorodi-thioate | Prothiofos |

N-Halogenothioalkyl Compounds:

| Compound No. | Compound Name | Common Name |
|---|---|---|
| N-1 | N-(Trichloromethylthio)cyclohex-4-ene-1,2-dicarboximide | Captan |
| N-2 | N-(1,1,2,2-Tetrachloroethylthio)cyclohex-4-ene-1,2-dicarboximide | Captafol |
| N-3 | N-(Trichloromethylthio)phthalimide | Folpet |

Dicarboximide Compounds:

| Compound No. | Compound Name | Common Name |
|---|---|---|
| O-1 | N-(3,5-Dichlorophenyl)-1,2-dimethylcyclopropane-1,2-dicarboximide | Procymidone |
| O-2 | 3-(3,5-Dichlorophenyl)-N-isopropyl-2,4-dioxoimidazol-idine-1-carboxamide | Iprodione |

| | | |
|---|---|---|
| O-3 | (RS)-3-(3,5-Dichlorophenyl)-5-methyl-5-vinyl-1,3-oxazolidine-2,4-dione | Vinclozolin |

<u>Benzanilide Compounds:</u>

| Compound No. | Compound Name | Common Name |
|---|---|---|
| P-1 | α,α,α-Trifluoro-3′-isopropoxy-o-toluanilide | Flutolanil |
| P-2 | 3′-Isopropoxy-o-toluanilide | Mepronil |

<u>Benzamide Compounds:</u>

| Compound No. | Compound Name | Common Name |
|---|---|---|
| Q-1 | 2-(1,3-Dimethylpyrazol-4-yl-carbonylamino)-4-methyl-3-pentenenitrile | disclosed in British Patent 2,190,375 |
| Q-2 | α-(Nicotinylamino)-(3-fluorophenyl)acetonitrile | disclosed in JP-A-63-135364 |

<u>Piperazine Compounds:</u>

| Compound No. | Compound Name | Common Name |
|---|---|---|
| R-1 | N,N′-[Piperazine-1,4-diylbis[(trichloromethyl)-methylene]]diformamide | Triforine |

Pyridine Compounds:

| Compound No. | Compound Name | Common Name |
|---|---|---|
| S-1 | 2',4'-Dichloro-2-(3-pyridyl)acetophenone O-methyloxime | Pyrifenox |

Pyrimidine Compounds:

| Compound No. | Compound Name | Common Name |
|---|---|---|
| T-1 | (±)-2,4'-Dichloro-α-(pyrimidin-5-yl)benzhydryl alcohol | Fenarimol |

Piperidine Compounds:

| Compound No. | Compound Name | Common Name |
|---|---|---|
| U-1 | (RS)-1-[3-(4-tert-Butylphenyl)-2-methylpropyl]-piperidine | Fenpropidin |

Morpholine Compounds:

| Compound No. | Compound Name | Common Name |
|---|---|---|
| V-1 | (±)-cis-4-[3-(4-tert-Butylphenyl)-2-methylpropyl]-2,6-dimethylmorpholine | Fenpropimorph |

Organotin Compounds:

| Compound No. | Compound Name | Common Name |
|---|---|---|
| W-1 | Triphenyltin hydroxide | Fentin Hydroxide |
| W-2 | Triphenyltin acetate | Fentin Acetate |

Urea Compounds:

| Compound No. | Compound Name | Common Name |
|---|---|---|
| X-1 | 1-(4-Chlorobenzyl)-1-cyclopentyl-3-phenylurea | Pencycuron |

Cinnamic Acid Compounds:

| Compound No. | Compound Name | Common Name |
|---|---|---|
| Y-1 | (E,Z)4-[3-(4-Chlorophenyl)-3-(3,4-dimethoxyphenyl)-acryloyl]morpholine | Dimethomorph |

Carbamate Compounds:

| Compound No. | Compound Name | Common Name |
|---|---|---|
| Za-1 | 1-Naphthyl methylcarbamate | Carbaryl |
| Za-2 | S-Methyl N-(methylcarbamoyloxy)thioacetimidate | Methomyl |
| Za-3 | 2-Ethylthiomethylphenyl methylcarbamate | Ethiofencarb |

| | | |
|---|---|---|
| Za-4 | Ethyl N-benzyl-N-[[methyl-[[(Z)-1-methylthioethylidene]aminooxycarbonyl]amino]thio]-β-alaninate | ORION® (trade name) |
| Za-5 | Isopropyl 3,4-diethoxycarbanilate | Diethofencarb |

Pyrethroid Compounds:

| Compound No. | Compound Name | Common Name |
|---|---|---|
| Zb-1 | (RS)-α-Cyano-3-phenoxybenzyl (Z)-(1RS,3RS)-(2-chloro-3,3,3-trifluoropropenyl)-2,2-dimethylcyclopropanecarboxylate | Cyhalothrin |
| Zb-2 | 2,3,5,6-Tetrafluoro-4-methylbenzyl (Z)-(1RS,3RS)-3-(2-chloro-3,3,3-trifluoroprop-1-enyl)-2,2-dimethylcyclopropanecarboxylate | Tefluthrin |
| Zb-3 | 2-(4-Ethoxyphenyl)-2-methylpropyl-3-phenoxybenzyl ether | Ethofenprox |
| Zb-4 | (RS)-α-Cyano-3-phenoxybenzyl (1RS,3RS; 1RS,3SR)-3-(2,2-dichlorovinyl)-2,2-dimethylcyclopropanecarboxylate | Cypermethrin |

Benzoylurea Compounds:

| Compound No. | Compound Name | Common Name |
|---|---|---|
| Zc-1 | 1-(4-Chlorophenyl)-3-(2,6-difluorobenzoyl)urea | Diflubenzuron |

| | | |
|---|---|---|
| Zc-2 | 1-(3,5-Dichloro-2,4-difluorophenyl)-3-(2,6-difluorobenzoyl)urea | Teflubenzuron |
| Zc-3 | 1-[3,5-Dichloro-4-(3-chloro-5-trifluoromethyl-2-pyridyloxy)phenyl]-3-(2,6-difluorobenzoyl)urea | Chlorfluazuron |

Thiazolidine Compounds:

| Compound No. | Compound Name | Common Name |
|---|---|---|
| Zd-1 | (4RS,5RS)-5-(4-Chlorophenyl)-N-cyclohexyl-4-methyl-2-oxo-1,3-thiazolidine-3-carboxamide | Hexythiazox |

Thiadiazine Compounds:

| Compound No. | Compound Name | Common Name |
|---|---|---|
| Ze-1 | 2-tert-Butylimino-3-isopropyl-5-phenyl-1,3,5-thiadiazinan-4-one | Buprofezin |

Nereistoxin Derivatives:

| Compound No. | Compound Name | Common Name |
|---|---|---|
| Zf-1 | N,N-Dimethyl-1,2,3-trithian-5-ylamine | Thiocyclam |
| Zf-2 | S,S′-2-Dimethylaminotrimethylene bis(thiocarbamate) | Cartap |

Pyridazinone Compounds:

| Compound No. | Compound Name | Common Name |
| --- | --- | --- |
| Zg-1 | 2-tert-Butyl-5-(4-tert-butylbenzylthio)-4-chloro-pyridazin-3(2H)-one | disclosed in European Patent 134,439A |

Spores of Bacillus thuringiensis and Crystalline Toxin Produced Thereby:

| Compound No. | Compound Name | Common Name |
| --- | --- | --- |
| Zh-1 | Bacillus thuringiensis subsp., kurstaki, HD-1 | THURICIDE® (trade name) |

Of the above-mentioned chemicals which can be used in combination with the compound of the present invention, preferred are pyrimidinamine compounds, azole compounds, quinoxaline compounds, dithiocarbamate compounds, organic chlorine compounds, benzimidazole compounds, pyridinamine compounds, cyanoacetamide compounds, phenylamide compounds, sulfenic acid compounds, copper compounds, isoxazole compounds, organophosphorus compounds, dicarboximide compounds, benzanilide compounds, benzamide compounds, pyridine compounds, pyrimidine compounds, morpholine compounds, and carbmate compounds. More preferred are Compound Nos. A-1, B-1 (Triadimefon), B-2 (Bitertanol), B-3 (Triflumizole), B-5 (Propiconazole), B-8 (Myclobutanil), B-11 (Hexaconazole), C-1 (Chinomethionate), D-1 (Maneb), D-2 (Zineb), D-3 (Mancozeb), E-2 (Chlorothalonil), F-1 (Benomyl), F-2 (Thiophanate-Methyl), G-1 (Fluazinam), I-1 (Metalaxyl), I-2 (oxadixyl), K-2 (Oxine-Copper), M-1 (Fosetyl-Al), 0-1 (Procymidone), 0-2 (Iprodione), 0-3 (Vinclozolin), S-1 (Pyrifenox), T-1 (Fenarimol), V-1 (Fenpropimorph), and Za-5 (Diethofencarb). In particular, Compound Nos. B-3 (Triflumizole), C-1 (Chinomethionate), D-3 (Mancozeb), E-2 (Chlorothalonil), F-1 (Benomyl), G-1 (Fluazinam), and 0-1 (Procymidone) are the most preferred.

A suitable mixing ratio of the compound of the present invention to these other chemicals usually ranges from 1:300 to 300:1, preferably from 1:100 to 100:1, and more preferably from 1:50 to 50:1, by weight.

The compounds according to the present invention also exhibit excellent antimicrobial activity on fungus, e.g., Trichophyton mentagraphytes, Trichophyton rubrum, Penicillium citrinum, and Aspergillus niger, as demonstrated in the antimicrobial susceptibility test as hereinafter described, and are therefore useful as medical and industrial antimicrobial agents.

In applying the compound of the present invention to humans for the purpose of treatment or prevention, it is formulated into various dose forms for external or internal use either alone or in combination with pharmaceutically acceptable additives, such as vehicles, diluents, and dispersing agents. Suitable dose forms include solutions, suspensions, powders, granules, capsules, tablets, ointments, creams, lotions, tinctures, etc. These preparations are generally prepared according to usual manner for medical preparations.

For administration in the form of an injectable solution or suspension, the compound of the present invention is administered at a dose level of from about 1 to 100 mg/kg-b.d. in about 2 to 4 divided doses per day. The dose per day depends on the step and degree of the infectious disease, susceptibility of the infectious microorganism to the chemical, characteristics of the patient, and the like.

In the case of oral administration, the compound of the invention is administered in a suitable dose form, such as tablets and capsules, at a dose of from 100 to 2,000 mg/day (reduced to the active ingredient) for an adult.

For external use, the compound of the invention is formulated into ointments, lotions, creams, etc. usually containing from about 0.1 to 10 g of the compound per 100 g and applied to an affected part about twice to five times per day.

Test Examples for evaluating the compounds of the present invention as agricultural and horticultural fungicides are hereinafter described. Evaluations were made according to the following standards unless otherwise specified.

Standards for Evaluation:

The controlling effect was determined by visually observing a degree of a disease of a test plant and expressed in terms of index of control according to the following rating system.

| Index of Control | Degree of Disease |
|---|---|
| 5 | No lesion is noted at all. |
| 4 | Area, number or length of lesions is less than 10% of that of the non-treated plot. |
| 3 | Area, number or length of lesions is less than 40% of that of the non-treated plot. |
| 2 | Area, number or length of lesions is less than 70% of that of the non-treated plot. |
| 1 | Area, number or length of lesions is 70% or more of that of the non-treated plot. |

TEST EXAMPLE 1

Test on Preventive Effect Against Cucumber Powdery Mildew

Cucumber (cultivars: Suyo) was cultivated in a polyethylene pot having a diameter of 7.5 cm. When the plant reached the one-leaf stage, 10 mℓ of a solution containing each of test compounds in a predetermined concentration was sprayed over the plant using a spray gun. After keeping the pots in a constant temperature chamber of 22° to 24°C for 7 to 24 hours, conidia of fungi of powdery mildew (*Sphaerotheca fuliginea*) were inoculated. Eight or nine days after the inoculation, an area of lesion on the first leaf was investigated to obtain an index of control according to the standards described above. The results obtained are shown in Table 4 below.

TABLE 4

| Compound No. | Index of Control 500 ppm |
|---|---|
| 1 | 5 |
| 3 | 5 |
| 5 | 4 |
| 10 | 5 |
| 50 | 5 |
| 52 | 4 |

TEST EXAMPLE 2

Test on Preventive Effect Against Cucumber Anthracnose

Cucumber (cultivars: Suyo) was cultivated in a polyethylene pot having a diameter of 7.5 cm. When the plant reached the two-leaf stage, 10 mℓ of a solution containing each of test compounds in a predetermined concentration was sprayed over the plant using a spray gun. After keeping the pots in a constant temperature chamber of 22° to 24° C for 24 hours, a spore suspension of fungi of anthracnose (*Colletotrichum lagenarium*) was inoculated. Five to seven days after the inoculation, an area of lesion on the first leaf was investigated to obtain an index of control according to the standards described above. The results obtained are shown in Table 5 below.

TABLE 5

| Compound No. | Index of Control | |
|---|---|---|
| | 500 ppm | 250 ppm |
| 1 | 5 | - |
| 3 | 5 | - |
| 4 | - | 4 |
| 5 | 5 | - |
| 7 | 5 | - |
| 9 | 4 | - |
| 10 | 5 | - |
| 13 | 5 | - |
| 14 | 4 | - |
| 16 | 5 | - |
| 21 | 5 | - |
| 22 | 4 | - |
| 24 | 5 | - |
| 41 | 5 | - |
| 46 | 4 | - |
| 49 | 4 | - |
| 52 | 5 | - |
| 53 | 5 | - |
| 77 | 5 | - |

TEST EXAMPLE 3

Test on Curative Effect on Cucumber Anthracnose

Cucumber (cultivars: Suyo) was cultivated in a polyethylene pot having a diameter of 7.5 cm. When the plant reached the two-leaf stage, a spore suspension of fungi of anthracnose (Colletotrichum lagenarium) was inoculated. After keeping the pots in a constant temperature chamber of 22° to 24°C for 24 hours in a humid condition, 10 mℓ of a solution containing each of test compounds in a predetermined concentration was sprayed over the plant using a spray gun. Four to seven days after the inoculation, an area of lesion on the first leaf was investigated to obtain an index of control according to the standards described above. The results obtained are shown in Table 6 below.

TABLE 6

| Compound No. | Index of Control 500 ppm |
|---|---|
| 1 | 5 |
| 9 | 5 |
| 10 | 5 |
| 22 | 5 |

TEST EXAMPLE 4

Test on Preventive Effect Against Cucumber Downy Mildew

Cucumber (cultivars: Suyo) was cultivated in a polyethylene pot having a diameter of 7.5 cm. When the plant reached the two-leaf stage, 10 mℓ of a solution containing each of test compounds in a predetermined concentration was sprayed over the plant using a spray gun. After keeping the pots in a constant temperature chamber of 22° to 24°C for 7 to 24 hours, a spore suspension of fungi of downy mildew (Pseudoperonospora cubensis) was inoculated. Five to eight days after the inoculation, an area of lesion on the first leaf was investigated to obtain an index of control according to the standards described above. The results obtained are shown in Table 7 below.

TABLE 7

| Compound No. | Index of Control 500 ppm |
|---|---|
| 1 | 5 |
| 2 | 4 |
| 13 | 5 |
| 26 | 4 |
| 50 | 5 |
| 53 | 5 |

TEST EXAMPLE 5

Test on Preventive Effect Against Rice Blast

Rice plant (cultivars: Koshihikari) was cultivated in a polyethylene pot having a diameter of 7.5 cm. When rice plant reached the four-leaf stage, 20 mℓ of a solution containing each test compound in a predetermined concentration was sprayed over the plant using a spray gun. After keeping the pots in a constant temperature chamber of 22° to 24° C for 7 to 24 hours, a spore suspension of fungi of blast (Pyricularia oryzae) was inoculated by spraying. Five to seven days after the inoculation, a number of lesions was investigated to obtain an index of control according to the above-described standards. The results obtained are shown in Table 8 below.

TABLE 8

| Compound No. | Index of Control 500 ppm |
|---|---|
| 1 | 5 |
| 3 | 4 |
| 4 | 4 |
| 5 | 4 |
| 7 | 4 |
| 8 | 4 |
| 9 | 5 |
| 10 | 5 |
| 13 | 5 |
| 16 | 5 |
| 22 | 5 |
| 41 | 5 |
| 54 | 4 |

TEST EXAMPLE 6

Test on Preventive Effect Against Rice Sheath Blight

Rice plant (cultivars: Koshihikari) was cultivated in a polyethylene pot having a diameter of 7.5 cm. When rice plant reached the five-leaf stage, 20 mℓ of a solution containing each test compound in a predetermined concentration was sprayed over the plant using a spray gun. After keeping the pots in a constant temperature chamber of 22° to 24° C for 7 to 48 hours, rice straw in which fungi of sheath blight (Rhizoctonia solani) had been previously incubated was set between leaf sheath portions to inoculate. After keeping the pots in an inoculation chamber at a temperature of 28° C and a humidity of 100% for 4 to 7 days, a length of lesion was investigated to obtain an index of control according to the standards described above. The results obtained are shown in Table 9 below.

TABLE 9

| Compound No. | Index of Control 500 ppm |
|---|---|
| 1 | 5 |
| 2 | 5 |
| 3 | 5 |
| 4 | 5 |
| 5 | 5 |
| 7 | 5 |
| 8 | 5 |
| 9 | 5 |
| 10 | 5 |
| 13 | 5 |
| 14 | 5 |
| 15 | 4 |
| 16 | 5 |
| 47 | 4 |
| 50 | 4 |
| 53 | 5 |

TEST EXAMPLE 7

Test on Preventive Effect Against Cucumber Gray Mold

Cucumber (cultivars: Suyo) was cultivated in a polyethylene pot having a diameter of 7.5 cm. When the plant reached the two-leaf stage, 10 mℓ of a solution containing each of test compounds in a predetermined concentration was sprayed over the plant using a spray gun. After keeping the pots in a constant temperature chamber at 22° to 24° C for 7 to 48 hours, the first leaf was inoculated with a mycelial disc (diameter: 5 mm) of fungi of gray mold (*Botrytis cinerea*) sensitive to benomyl and dicarboximide or resistant to benomyl and dicarboximide. Three to four days after the inoculation, an length of lesion was investigated to obtain an index of control according to the standards described above. The results obtained are shown in Tables 10 and 11 below.

TABLE 10

Benomyl- and Dicarboximide-Sensitive Strain

| Compound No. | Index of Control 500 ppm |
|---|---|
| 1 | 5 |
| 3 | 5 |
| 5 | 4 |
| 7 | 5 |
| 9 | 4 |
| 10 | 4 |
| 15 | 4 |
| 16 | 5 |
| 19 | 4 |
| 21 | 5 |
| 22 | 5 |
| 24 | 5 |
| 41 | 5 |
| 52 | 5 |
| 53 | 5 |

TABLE 11

Benomyl- and Dicarboximide-Resistant Strain

| Compound No. | Index of Control 500 ppm |
|---|---|
| 1 | 5 |
| 3 | 5 |
| 4 | 4 |
| 5 | 4 |
| 8 | 5 |
| 9 | 4 |
| 10 | 5 |
| 16 | 4 |
| 22 | 5 |
| 24 | 5 |
| 26 | 5 |
| 41 | 4 |
| 49 | 5 |
| 52 | 5 |
| 53 | 5 |

## TEST EXAMPLE 8

### Test on Curative Effect on Cucumber Gray Mold

Cucumber (cultivars: Suyo) was cultivated in a polyethylene pot having a diameter of 7.5 cm. When the plant reached the two-leaf stage, the first leaf was inoculated with a mycelial disc (diameter: 5 mm) of fungi of gray mold (Botrytis cinerea) sensitive to benomyl and dicarboximide. After 1 day from the inoculation, the inoculated leaf was immersed in a 1,000 ppm solution of Compound No. 1. One day after the immersion, a length of lesion was investigated, and a control value was obtained according to equation:

$$\text{Control Value} = \left(1 - \frac{a - c}{b - c}\right) \times 100$$

wherein a is a length of lesion of the treated plot; b is a length of lesion of the non-treated plot; and c is a length of lesion before the treatment.

As a result, the control value after 1 day from the immersion was found to be 67.

TEST EXAMPLE 9

Test on Preventive Effect on Oats Crown Rust

Oats (cultivars: Zenshin) was cultivated in a polyethylene pot having a diameter of 7.5 cm. When oats reached the two-leaf stage, 10 mℓ of a 500 ppm solution of each test compound was sprayed over the plant using a spray gun. After keeping the pots in a constant temperature chamber at 22° to 24°C for 7 to 24 hours, conidia of fungi of crown rust (Puccinia coronata) were inoculated. Twelve to fourteen days after the inoculation, an area of lesion on the second leaf was investigated to obtain an index of control according to the standards described above. The results obtained are shown in Table 12 below.

TABLE 12

| Compound No. | Index of Control 500 ppm |
|---|---|
| 9 | 5 |
| 10 | 5 |
| 19 | 5 |
| 41 | 5 |

TEST EXAMPLE 10

Test on Preventive Effect Against Cucumber Anthracnose

Cucumber (cultivars: Suyo) was cultivated in a polyethylene pot having a diameter of 7.5 cm. When the plant reached the two-leaf stage, 10 mℓ of a solution containing each test compound or compounds in a predetermined concentration(s) was sprayed over the plant using a spray gun. After keeping the pots in a constant temperature chamber of 22° to 24°C for one day, a spore suspension of fungi of anthracnose (Colletotrichum lagenarium) was inoculated by spraying. Six days after the inoculation, a number of lesion on the first leaf was investigated. The results obtained are shown in Tables 13 and 14 below.

TABLE 13

| Compound No. | Concentration (ppm) | Number of Lesions |
|---|---|---|
| 1 | 63 | 38 |
| O-1 | 63 | 70 |
| mixture of 1 and O-1 | 63 + 63 | 2 |

TABLE 14

| Compound No. | Concentration (ppm) | Number of Lesions |
|---|---|---|
| 1 | 31 | 40 |
| C-1 | 63 | 70 |
| mixture of 1 and C-1 | 31 + 63 | 7 |

TEST EXAMPLE 11

Test on Preventive Effect Against Cucumber Gray Mold

Cucumber (cultivars: Suyo) was cultivated in a polyethylene pot having a diameter of 7.5 cm. When the plant reached the two-leaf stage, 10 mℓ of a solution containing each test compound or compounds in a predetermined concentration(s) was sprayed over the plant using a spray gun. After keeping the pots in a constant temperature chamber at 22° to 24° C for 24 to 48 hours, the first leaf was inoculated with a mycelial disc (diameter: 5 mm) of fungi of gray mold (Botrytis cinerea) sensitive to benomyl and dicarboximide. Three days after the inoculation, an length of lesion was investigated. The results obtained are shown in Tables 15 through 19 below.

TABLE 15

| Compound No. | Concentration (ppm) | Length of Lesions (mm) |
|---|---|---|
| 1 | 8 | 32 |
| B-3 | 125 | 18 |
| mixture of 1 and B-3 | 8 + 125 | 4 |

TABLE 16

| Compound No. | Concentration (ppm) | Length of Lesions (mm) |
|---|---|---|
| 1 | 16 | 15 |
| D-3 | 16 | 36 |
| mixture of 1 and D-3 | 16 + 16 | 0 |

TABLE 17

| Compound No. | Concentration (ppm) | Length of Lesions (mm) |
|---|---|---|
| 1 | 4 | 30 |
| F-1 | 16 | 15 |
| mixture of 1 and F-1 | 4 + 16 | 4 |

TABLE 18

| Compound No. | Concentration (ppm) | Length of Lesions (mm) |
|---|---|---|
| 1 | 31 | 41 |
| G-1 | 4 | 44 |
| mixture of 1 and G-1 | 31 + 4 | 10 |

TABLE 19

| Compound No. | Concentration (ppm) | Length of Lesions (mm) |
|---|---|---|
| 1 | 16 | 29 |
| O-1 | 16 | 21 |
| mixture of 1 and O-1 | 16 + 16 | 2 |

TEST EXAMPLE 12

Test on Preventive Effect on Cucumber Downy Mildew

Cucumber (cultivars: Suyo) was cultivated in a polyethylene pot having a diameter of 7.5 cm. When the plant reached the two-leaf stage, 10 mℓ of a solution containing each test compound or compounds in a predetermined concentration(s) was sprayed over the plant using a spray gun. After keeping the pots in a constant temperature chamber of 22° to 24° C for one day, a spore suspension of fungi of downy mildew (Pseudoperonospora cubensis) was inoculated. Six days after the inoculation, an area of lesion on the first leaf was investigated to obtain an index of control according to the standards described above. The results obtained are shown in Table 20 below.

TABLE 20

| Compound No. | Concentration (ppm) | Index of Control (%) |
|---|---|---|
| 1 | 8 | 2 |
| E-2 | 16 | 3 |
| mixture of 1 and E-2 | 8 + 16 | 5 |

Test Examples for evaluating the compounds of the present invention as agricultural and horticultural insecticides are hereinafter described.

TEST EXAMPLE 13

Test on Miticidal Effect on Adults of Two-Spotted Spider Mites

Kidney bean (cultivars: Edogawa Saito) with only one primary leaf left was transplanted on a polyethylene pot having a diameter of 7.5 cm. About 30 adults of two-spotted spider mites (Tetranychus urticae) having resistance to Dicofol and organophosphorus compounds were released on the leaf, and the infested leaf was immersed in 20 mℓ of a 800 ppm solution of each test compound for about 10 seconds. After air drying, the plant was kept in a constant temperature chamber of 26°C with lighting. Five to seven days after the treatment, numbers of dead mites and live mites were investigated to obtain a mortality (%) according to equation:

$$\text{Mortality (\%)} = \frac{\text{Number of Dead Mites}}{\text{Number of Released Mites}} \times 100$$

As a result, Compound Nos. 1, 4, 9, 10, and 16 each showed a mortality of 100%.

TEST EXAMPLE 14

Test on Ovicidal Effect on Two-Spotted Spider Mites

Kidney bean (cultivars: Edogawa Saito) with only one primary leaf left was transplanted on a polyethylene pot. Thirty adults of two-spotted spider mites (Tetranychus urticae) were released thereon and allowed to oviposit for 24 hours. After removing the adults of mites, the plant was immersed in 20 mℓ of a 800 ppm solution of each test compound for about 10 seconds and air-dried. The plant was kept in a constant temperature chamber of 26°C with lighting. Five to seven days after the treatment, a state of hatching was investigated to obtain an ovicidal rate (%) according to equation:

$$\text{Ovicidal Rate (\%)} = \frac{\text{Number of Killed Eggs}}{\text{Number of Oviposited Eggs}} \times 100$$

Death immediately after hatching was regarded to be ovicide. As a result, Compound Nos. 1, 4, 7, 9, 10, and 16 each showed an ovicidal rate of 100%.

TEST EXAMPLE 15

Test on Insecticidal Effect on Small Brown Planthopper

Young seedlings of rice plant (cultivars: Nihonbare) were immersed in 20 mℓ of a 800 ppm solution of each test compound for about 10 seconds. After air-drying, the root was wrapped with wet absorbent cotton and put in a test tube. Ten larvae of second to third instar of small brown planthopper (Laodelphax striatellus) were released in the test tube, and the opening of the test tube was covered with gauze. The test tube was kept in a constant temperature chamber of 26°C with lighting. Five days after the release of the larvae, a number of dead insects was investigated to obtain a mortality according to the equation described in Test Example 13. As a result, Compound No. 9 showed a mortality was 100%.

TEST EXAMPLE 16

Test on Insecticidal Effect on Green Peach Aphids

Egg plant (cultivars: Senryo Ni-Go) with only one leaf left was transplanted in a cup having a diameter of 8 cm and a height of 7 cm, and 2 to 3 apterous viviparous females of green peach aphids (Myzus persicae) were released on the leaf and allowed to oviposit. Two days after the release of the insects, the adult insects were removed, and a number of larvae was counted. The leaf infested with the larvae was immersed in a 800 ppm aqueous dispersion of each test compound for about 10 seconds. After air-drying,

the plant was kept in a constant temperature chamber of 26° C with lighting. Five days after the release of the insects, a number of dead insects was investigated. The insects having dropped off the leaf were regarded dead. As a result, Compound Nos. 1, 9 and 10 showed a mortality of 100% as determined in the same manner as in Test Example 13.

Test Examples for evaluating the compounds of the present invention as medical and industrial antimicrobial agents are hereinafter described.

TEST EXAMPLE 17

Test on Antimicrobial Activity

Antimicrobial susceptibility tests were carried out using fungus (a), (b), (c) and (d) on Sabouraud agar medium to obtain minimal inhibitory concentration (MIC). The results obtained are shown in Table 21 below.

(a) *Trichophyton mentagraphytes* IFO-5809
(b) *Trichophyton rubrum* IFO-9158
(c) *Penicillium citrinum* IFO-6352
(d) *Aspergillus niger* IFO-6342

TABLE 21

| Compound No. | MIC (ppm) (a) | (b) | (c) | (d) |
|---|---|---|---|---|
| 1 | 3.13 | 3.13 | 0.20 | 0.39 |
| 2 | 0.39 | 3.13 | 6.25 | 1.56 |
| 7 | 12.5 | 12.5 | 0.78 | 3.13 |
| 52 | 25 | 12.5 | 0.78 | 0.78 |

Formulation Examples of the compounds according to the present invention are described below, but it should be understood that the present invention is not deemed to be limited thereto.

FORMULATION EXAMPLE 1

| | | |
|---|---|---|
| (a) | Compound No. 1 | 50 parts by weight |
| (b) | Kaolin | 40 parts by weight |
| (c) | Sodium lignin sulfonate | 7 " |
| (d) | Dialkyl sulfosuccinate | 3 " |

The above components were uniformly mixed to obtain a wettable powder.

FORMULATION EXAMPLE 2

| | | |
|---|---|---|
| (a) | Compound No. 3 | 20 parts by weight |
| (b) | White carbon (hydrated amorphous silicon dioxide) | 20 " |
| (c) | Kaolin | 52 " |
| (d) | Sodium lignin sulfonate | 4 " |
| (e) | Polyoxyethylene alkylaryl ether | 4 " |

The above components were uniformly mixed to obtain a wettable powder.

FORMULATION EXAMPLE 3

| | | |
|---|---|---|
| (a) | Kaolin | 78 parts by weight |
| (b) | Sodium β-naphthalenesulfonate-formalin condensate | 2 " |
| (c) | Polyoxyethylene alkylaryl sulfate | 5 " |
| (d) | White carbon (hydrated amorphous silicon dioxide) | 15 " |

A mixture of the above components and Compound No. 9 were mixed at a weight ratio of 4:1 to obtain a wettable powder.

FORMULATION EXAMPLE 4

| | | |
|---|---|---|
| (a) | Compound No. 10 | 10 parts by weight |
| (b) | Diatomaceous earth | 69 " |
| (c) | Calcium carbonate powder | 15 " |
| (d) | Dialkyl sulfosuccinate | 1 part by weight |
| (e) | Polyoxyethylene alkylphenyl ether sulfate | 3 " |
| (f) | Sodium β-naphthalenesulfonate-formalin condensate | 2 " |

The above components were uniformly mixed to obtain a wettable powder.

FORMULATION EXAMPLE 5

| | | |
|---|---|---|
| (a) | Compound No. 22 | 10 parts by weight |
| (b) | Diatomaceous earth | 45 " |
| (c) | Calcium carbonate powder | 39 " |
| (d) | Dialkyl sulfosuccinate | 1 " |
| (e) | Polyoxyethylene alkylphenyl ether sulfate | 3 " |
| (f) | Sodium β-naphthalenesulfonate-formalin condensate | 2 " |

The above components were uniformly mixed to obtain a wettable powder.

FORMULATION EXAMPLE 6

| | | |
|---|---|---|
| (a) | Compound No. 50 | 6 parts by weight |
| (b) | Diatomaceous earth | 88 " |
| (c) | Dialkyl sulfosuccinate | 2 " |
| (d) | Polyoxyethylene alkylphenyl ether sulfate | 4 " |

The above components were uniformly mixed to obtain a wettable powder.

FORMULATION EXAMPLE 7

| | | |
|---|---|---|
| (a) | Compound No. 53 | 0.5 part by weight |
| (b) | Talc | 99.0 parts by weight |
| (c) | Lower alcohol phosphate | 0.5 " |

The above components were uniformly mixed to obtain a dust.

FORMULATION EXAMPLE 8

| | | |
|---|---|---|
| (a) | Compound No. 3 | 0.2 part by weight |
| (b) | Calcium carbonate powder | 98.8 " |
| (c) | Lower alcohol phosphate | 1.0 " |

The above components were uniformly mixed to obtain a dust.

FORMULATION EXAMPLE 9

| | | |
|---|---|---|
| (a) | Compound No. 57 | 20 parts by weight |
| (b) | Xylene | 60 " |
| (c) | Polyoxyethylene alkylaryl ether | 20 " |

The above components were mixed and dissolved to obtain an emulsifiable concentrate.

FORMULATION EXAMPLE 10

| | | |
|---|---|---|
| (a) | Compound No. 10 | 1 part by weight |
| (b) | Bentonite | 30 " |
| (c) | Kaolin | 64 " |
| (d) | Sodium lignin sulfonate | 5 " |

The above components were mixed with a requisite amount of water and granulated to obtain granules.

FORMULATION EXAMPLE 11

| | | |
|---|---|---|
| (a) | Compound No. 1 | 40 parts by weight |
| (b) | Oxyethylated polyarylphenol phosphate | 3 " |
| (c) | Sodium methylnaphthalenesulfonate condensate | 3 " |
| (d) | Magnesium aluminum silicate | 1 part by weight |
| (e) | Ethylene glycol | 10 " |
| (f) | Xanthan gum | 0.06 " |
| (g) | Water | 42.94 " |

The above components were mixed and wet ground to obtain an aqueous suspension (flowable preparation).

FORMULATION EXAMPLE 12

| | | |
|---|---|---|
| (a) | Compound No. 1 | 5 parts by weight |
| (b) | Compound No. O-1 | 45 " |
| (c) | Kaolin | 40 " |
| (d) | Calcium lignin sulfonate | 7 " |
| (e) | Dialkyl sulfosuccinate | 3 " |

The above components were uniformly mixed to obtain a wettable powder.

FORMULATION EXAMPLE 13

| | | |
|---|---|---|
| (a) | Compound No. 1 | 5 parts by weight |
| (b) | Compound No. F-1 | 15 " |
| (c) | Calcium carbonate | 20 " |
| (d) | Kaolin | 52 " |
| (e) | Calcium lignin sulfonate | 4 " |
| (f) | Polyoxyethylene alkylaryl ether | 4 " |

The above components were uniformly mixed to obtain a wettable powder.

FORMULATION EXAMPLE 14

| | | |
|---|---|---|
| (a) | Kaolin | 78 parts by weight |
| (b) | Sodium β-naphthalenesulfonate-formalin condensate | 2 parts by weight |
| (c) | Polyoxyethylene alkylaryl sulfate | 5 " |
| (d) | White carbon (hydrated amorphous silicon dioxide) | 15 " |

A mixture of the above components, Compound No. 1, and Compound No. E-2 were mixed at a weight ratio of 8:1:1 to obtain a wettable powder.

FORMULATION EXAMPLE 15

| | | |
|---|---|---|
| (a) | Active ingredient (compound of invention) | 50 mg |
| (b) | Lactose fine powder | 197 mg |
| (c) | Corn starch | 30 mg |
| (d) | Magnesium stearate | 3 mg |

The above components were mixed and granulated, followed by sieving. The granules were charged in two-piece capsules of hardened gelatin by means of an appropriate capsulating machine to obtain capsules.

FORMULATION EXAMPLE 16

| | | |
|---|---|---|
| (a) | Ultra-finely divided active ingredient (compound of invention) | 20 mg |
| (b) | Mineral oil | 50 mg |
| (c) | White petrolatum | 930 mg |

Component (a) was dispersed in a part of the mineral oil (b). Component (c) and the rest of the mineral oil (b) were heated to 65° C and mixed until the mixture became homogeneous. The resulting mixture was cooled to 50° to 55° C while stirring, and the above-prepared mineral oil dispersion of the active ingredient (a) was added thereto with stirring. The resulting mixture was allowed to cool to room temperature to obtain an ointment.

While the invention has been described in detail and with reference to specific examples thereof, it will be apparent to one skilled in the art that various changes and modifications can be made therein without departing from the spirit and scope thereof.

**Claims**

**1.** A pyridylcarbamate compound represented by formula (I):

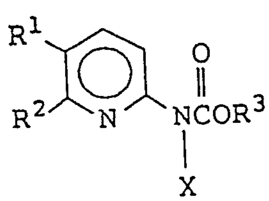

(I)

wherein $R^1$ represents a halogen atom, an alkoxy group, a trifluoromethyl group, a cyano group, or an alkylthio group; $R^2$ represents an alkyl group or an alkylthio group; $R^3$ represents a substituted or unsubstituted alkyl group, a substituted or unsubstituted alkenyl group, or a substituted or unsubstituted alkynyl group; and X represents a hydrogen atom, a substituted or unsubstituted alkyl group, or a substituted or unsubstituted acyl group; provided that a combination of $R^1$ representing a halogen atom, $R^2$ representing an alkyl group, $R^3$ representing an unsubstituted alkyl group or a tetrahalogenoethyl group, and X representing a hydrogen atom and a combination of $R^1$ representing an alkoxy group and $R^2$ representing an alkylthio group are excluded.

**2.** A compound according to Claim 1, wherein X represents a hydrogen atom.

3. A compound according to Claim 1, wherein $R^1$ represents a halogen atom; $R^2$ represents an alkyl group; $R^3$ represents a substituted or unsubstituted alkynyl group; and X represents a hydrogen atom.

4. A compound according to Claim 1, wherein $R^1$ represents an iodine atom; $R^2$ represents an ethyl group; $R^3$ represents a propargyl group; and X represents a hydrogen atom.

5. A compound according to Claim 1, wherein X represents a substituted or unsubstituted alkyl group or a substituted or unsubstituted acyl group.

6. A compound according to Claim 1, wherein $R^1$ represents an iodine atom; $R^2$ represents an ethyl group; $R^3$ represents a methyl group; and X represents an ethyl group.

7. A process for preparing a pyridylcarbamate compound represented by formula (I-a):

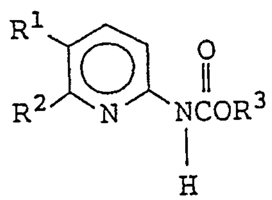

(I-a)

wherein $R^1$ represents a halogen atom, an alkoxy group, a trifluoromethyl group, a cyano group, or an alkylthio group; $R^2$ represents an alkyl group or an alkylthio group; and $R^3$ represents a substituted or unsubstituted alkyl group, a substituted or unsubstituted alkenyl group, or a substituted or unsubstituted alkynyl group; provided that a combination of $R^1$ representing a halogen atom, $R^2$ representing an alkyl group, and $R^3$ representing an unsubstituted alkyl group or a tetrahalogenoethyl group and a combination of $R^1$ representing an alkoxy group and $R^2$ representing an alkylthio group are excluded,
which comprises reacting a compound represented by formula (II):

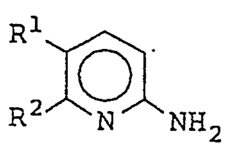

(II)

wherein $R^1$ and $R^2$ are as defined above,
with a compound represented by formula:

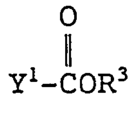

wherein $R^3$ is as defined above; and $Y^1$ represents a halogen atom.

8. A process for preparing a pyridylcarbamate compound represented by formula (I-b):

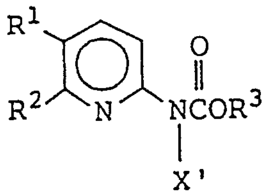

(I-b)

wherein $R^1$ represents a halogen atom, an alkoxy group, a trifluoromethyl group, a cyano group, or an alkylthio group; $R^2$ represents an alkyl group or an alkylthio group; $R^3$ represents a substituted or unsubstituted alkyl group, a substituted or unsubstituted alkenyl group, or a substituted or unsubstituted alkynyl group; provided that a combination of $R^1$ representing an alkoxy group and $R^2$ representing an alkylthio group is excluded; and X' represents a substituted or unsubstituted alkyl group, or a substituted or unsubstituted acyl group, which comprises reacting a compound represented by formula (I-a):

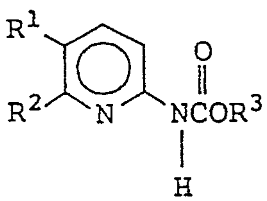

(I-a)

wherein $R^1$, $R^2$, and $R^3$ are as defined above,
with a compound represented by formula:

$$X' - Y^2$$

wherein X' is as defined above; and $Y^2$ represents a chlorine atom, a bromine atom, or an iodine atom.

**9.** A process for preparing a pyridylcarbamate compound represented by formula (I-d):

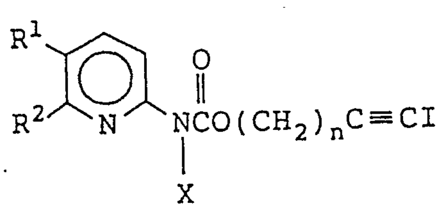

(I-d)

wherein $R^1$ represents a halogen atom, an alkoxy group, a trifluoromethyl group, a cyano group, or an alkylthio group; $R^2$ represents an alkyl group or an alkylthio group; provided that a combination of $R^1$ representing an alkoxy group and $R^2$ representing an alkylthio group is excluded; X represents a hydrogen atom, a substituted or unsubstituted alkyl group, or a substituted or unsubstituted acyl group; and n represents an integer of from 1 to 4,
which comprises reacting a compound represented by formula (I-c):

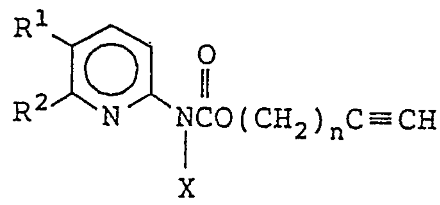

(I-c)

wherein $R^1$, $R^2$, X, and n are as defined above, with an iodinating agent.

**10.** A biocidal composition for controlling harmful organisms, which comprises a pyridylcarbamate compound represented by formula (I):

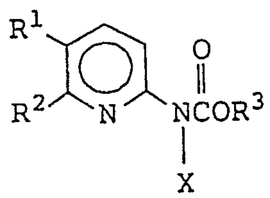

(I)

wherein $R^1$ represents a halogen atom, an alkoxy group, a trifluoromethyl group, a cyano group, or an alkylthio group; $R^2$ represents an alkyl group or an alkylthio group; $R^3$ represents a substituted or unsubstituted alkyl group, a substituted or unsubstituted alkenyl group, or a substituted or unsubstituted alkynyl group; and X represents a hydrogen atom, a substituted or unsubstituted alkyl group, or a substituted or unsubstituted acyl group; provided that a combination of $R^1$ representing a halogen atom, $R^2$ representing an alkyl group, $R^3$ representing an unsubstituted alkyl group or a tetrahalogenoethyl group, and X representing a hydrogen atom and a combination of $R^1$ representing an alkoxy group and $R^2$ representing an alkylthio group are excluded,
as an active ingredient and adjuvants.

**11.** A biocidal composition for controlling harmful organisms, which comprises at least one pyridylcarbamate compound represented by formula (I):

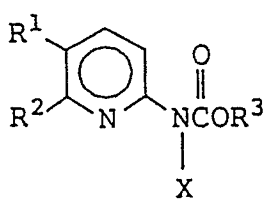

(I)

wherein $R^1$ represents a halogen atom, an alkoxy group, a trifluoromethyl group, a cyano group, or an alkylthio group; $R^2$ represents an alkyl group or an alkylthio group; $R^3$ represents a substituted or unsubstituted alkyl group, a substituted or unsubstituted alkenyl group, or a substituted or unsubstituted alkynyl group; and X represents a hydrogen atom, a substituted or unsubstituted alkyl group, or a substituted or unsubstituted acyl group; provided that a combination of $R^1$ representing a halogen atom, $R^2$ representing an alkyl group, $R^3$ representing an unsubstituted alkyl group or a tetrahalogenoethyl group, and X representing a hydrogen atom and a combination of $R^1$ representing an alkoxy group and $R^2$ representing an alkylthio group are excluded,
and at least one compound selected from a pyrimidinamine compound, an azole compound, a quinoxaline compound, a dithiocarbamate compound, an organic chlorine compound, a benzimidazole compound, a pyridinamine compound, a cyanoacetamide compound, a phenylamide compound, a

sulfenic acid compound, a copper compound, an isoxazole compound, an organophosphorus compound, an N-halogenothioalkyl compound, a dicarboximide compound, a benzanilide compound, a benzamide compound, a piperazine compound, a pyridine compound, a pyrimidine compound, a piperidine compound, a morpholine compound, an organotin compound, a urea compound, a cinnamic acid compound, a carbamate compound, a pyrethroid compound, a benzoylurea compound, a thiazolidine compound, a thiadiazine compound, a nereistoxin derivative, a pyridazinone compound, and spores of Bacillus thuringiensis and crystalline toxin produced thereby as active ingredients.